# EUROPEAN PATENT APPLICATION

(11) **EP 3 943 159 A1**
(43) Date of publication of application: **26.01.2022**
(21) Application number: 20778570.0
(22) Date of filing: 19.03.2020
(51) Int. Cl.: A61P 35/00, A61P 43/00, C07D 405/04, C07D 409/04, C07D 413/04, C07D 413/14, C07D 417/04, C07B 61/00, C07D 209/38, C07D 215/52, A61K 31/4709

(54) **PRODUCTION METHOD OF QUINOLINECARBOXAMIDE DERIVATIVE OR PRODUCTION INTERMEDIATE THEREOF**

(30) Priority: 22.03.2019 JP 2019055050; 22.03.2019 JP 2019055051; 22.03.2019 JP 2019055052
(71) Applicant: Kabushiki Kaisha Yakult Honsha, Tokyo, 105-8660 (JP)
(72) Inventor: SHIGEYAMA, Takahide, Tokyo 105-8660 (JP); SUGIMOTO, Takuya, Tokyo 105-8660 (JP); KOMATSU, Hideyuki, Tokyo 105-8660 (JP); NISHIYAMA, Hiroyuki, Tokyo 105-8660 (JP)
(74) Representative: Schiener, Jens
(86) International application number: PCT/JP2020/012477
(87) International publication number: WO 2020/196327

(57) **Abstract**

Provided is a method for industrially advantageously synthesizing a production intermediate of a quinolinecarboxamide derivative or a salt thereof. The present invention provides a method for producing a quinolinecarboxylic acid derivative of formula (4) or a salt thereof, including reacting an aniline of the following formula (1), in the presence of boron trifluoride-tetrahydrofuran complex or boron trifluoride-diethyl ether complex, with an aldehyde of formula (2) and subsequently reacting the resulting compound with an α-keto acid of formula (3), wherein R¹, R², R³ and R⁴ are the same or different and each represent a hydrogen atom, a halogen atom, a lower alkyl group, or the like, R⁵ represents a hydrogen atom, a lower alkyl group, or the like, and R⁶ represents a hydrogen atom, a lower alkyl group, or the like.

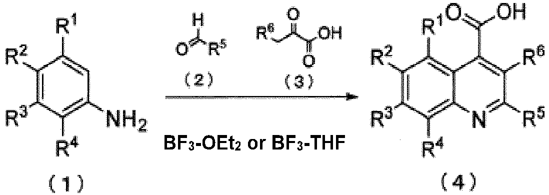

## Description

### Field of the Invention

The present invention relates to a method for producing a quinolinecarboxamide derivative or a production intermediate thereof.

### Background of the Invention

STAT (signal transducers and activators of transcription), a transcription factor, is a DNA binding protein and is deeply involved in cell growth, differentiation, etc. as an essential mediator for a pathway of signal transduction from cell surface to the nucleus. STAT is known to consist of 7 different families, among which STAT3 is found to be constantly activated and overexpressed in many cancer cells and involved in the growth or infiltration of the cancer cells. Thus, inhibitors of STAT3 are expected as anticancer agents. The present applicant has found specific quinolinecarboxamide derivatives including a compound of formula (A6) or formula (B8) given below (wherein R¹, R², R³, R⁴, R⁵ and R⁶ are as mentioned later)

### (Patent Literature 1).

The quinolinecarboxamide derivative of formula (A6) or (B8) is as described below.

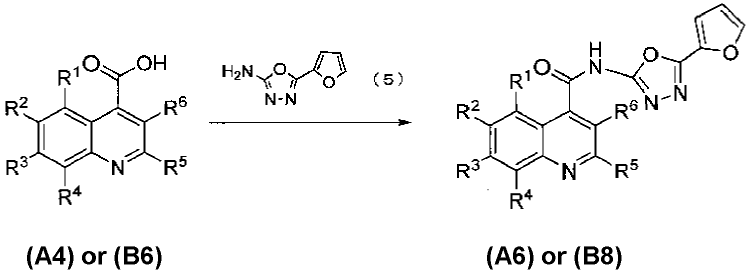

The compound is produced by reacting a quinolinecarboxylic acid derivative of formula (A4) or (B6) with 5-(furan-2-yl)-1,3,4-oxadiazol-2-amine. It has previously been reported that such a quinolinecarboxylic acid derivative of formula (A4) or (B6) is produced by a production method of reacting an aniline, an aldehyde or an α-keto acid in the presence or absence of an acid, as shown below (Non Patent Literatures A1 and A2): 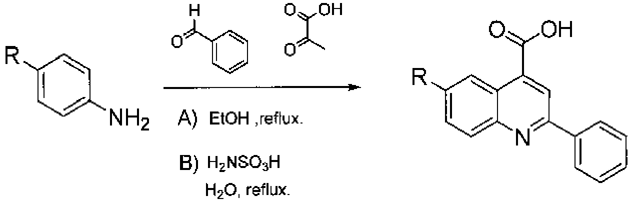 wherein R represents a hydrogen atom, a methyl group, a nitro group, or the like.

It is also known that such a quinolinecarboxylic acid derivative of formula (A4) or (B6) can be produced by use of a Pfitzinger quinoline synthesis method from a corresponding isatin derivative (Non Patent Literature B1). In this context, the isatin derivative for use as a starting material can generally be synthesized by cyclizing a product obtained by reacting aniline with hydrated chloral and hydroxylamine in sulfuric acid, as shown below (Non Patent Literature B2).

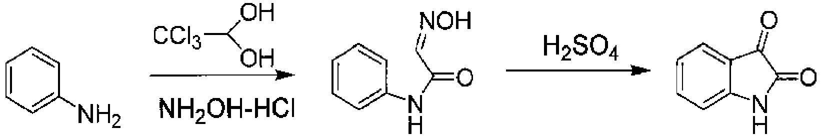

However, problems of this method are use of explosive hydroxylamine and the generation of by-products at the time of synthesis of an intermediate when the substrate aniline is used in a high concentration in producing the isatin derivative corresponding to the quinolinecarboxylic acid derivative of formula (A4) or (B6) .

Meanwhile, it has previously been reported that 5-(furan-2-yl)-1,3,4-oxadiazol-2-amine for use in the production of the quinolinecarboxamide derivative of formula (A6) or (B8) is produced by a production method of reacting furfural with semicarbazide to obtain semicarbazone, which is then isolated and then cyclized using iodine as an oxidizing agent, as shown below (Non Patent Literature C1).

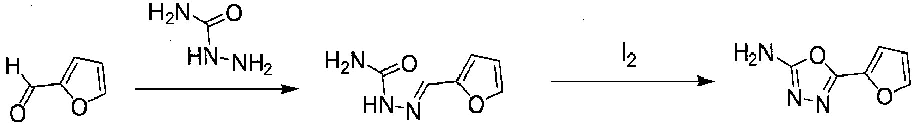

However, problems of this method are time-consuming operation due to the isolation of semicarbazone, use of highly toxic, corrosive and sublimating iodine, and low yields of the cyclization reaction.

### Citation List

### Patent Literature

Patent Literature 1: JP-B-5650529

### Non Patent Literature

Non Patent Literature A1: Synthetic comm., 2011, 41, 1435-1443
Non Patent Literature A2: Mod. Chem. Appl., 2016, 4, 195/1-195/6
Non Patent Literature B1: Heterocycles, 2014, 89, 693-707 Non Patent Literature B2: Bioorg. Med. Chem., 2010, 18, 1482-1496
Non Patent Literature C1: J. Org. Chem., 2015, 80, 1018-1024

### Summary of the Invention

### Technical Problem

First, the present invention relates to providing a method for industrially advantageously synthesizing a quinolinecarboxylic acid derivative of formula (A4) or a salt thereof which is a production intermediate of a quinolinecarboxamide derivative of formula (A6) or a salt thereof.

Secondly, the present invention relates to providing a method for industrially advantageously synthesizing an isatin derivative (formula (B4) given below) or a salt thereof which may be used as a starting material in the production of a quinolinecarboxamide derivative of formula (B8) or a salt thereof.

Thirdly, the present invention relates to providing a method for industrially advantageously synthesizing a 1,3,4-oxa(thia)diazol-2-amine derivative which may be used in the production of STAT3 inhibitors including a quinolinecarboxamide derivative of formula (A6) or (B8).

### Solution to Problem

The present inventors conducted diligent studies in light of these circumstances and consequently found that in the case of producing a quinolinecarboxylic acid derivative using an aniline, an aldehyde or an α-keto acid, a quinolinecarboxylic acid derivative of formula (A4) can be produced at high yields by using boron trifluoride-tetrahydrofuran complex or boron trifluoride-diethyl ether complex, as shown below, and by extension, a quinolinecarboxamide derivative of formula (A6) or a salt thereof can be efficiently produced:

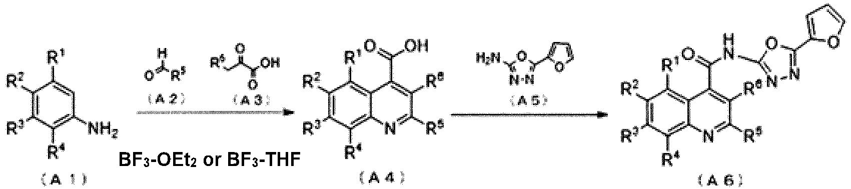

wherein R¹, R², R³ and R⁴ are the same or different and each represent a hydrogen atom, a halogen atom, a lower alkyl group, a nitro group, a hydroxy group, a cyano group, an optionally substituted phenyl group, an optionally substituted 5- or 6-membered heterocyclic group, a lower alkoxy group, a halo-lower alkoxy group, a lower alkoxy-lower alkoxy group, a lower alkyl-carbonyl group, a lower alkoxy-carbonyl group, a benzyloxy group, a trityloxy group, a t-butyldimethylsilyloxy group, a di-lower alkyl-amino group, a t-butoxycarbonylamino group, a benzyloxycarbonylamino group, a 2-nitrobenzenesulfonylamino group or a lower alkyl-thio group, R⁵ represents a hydrogen atom, a lower alkyl group, a cyclo-lower alkyl group, an optionally substituted phenyl group, an optionally substituted 5- or 6-membered heterocyclic group, a naphthyl group, a 1,3-benzodioxolyl group, a styryl group, or -CR^{5a}OR^{5b}CH₂OR^{5c} (wherein R^{5a} represents a methyl group or a t-butyl group, and R^{5b} and R^{5c} are the same or different and each represent a hydrogen atom, a lower alkyl group, a lower alkanoyl group, a t-butyldimethylsilyl group, a 2-(trimethylsilyl)ethoxymethyl group, a benzyloxymethyl group, a phenyl group or a benzyl group optionally having a substituent on the ring, a lower alkenyl group or a lower alkoxy-methyl group), and R⁶ represents a hydrogen atom, a lower alkyl group, a hydroxy group, an aryl group or a halogen atom.

Specifically, a first aspect of the present invention relates to the following 1) to 3).
1) A method for producing a quinolinecarboxylic acid derivative of formula (A4) or a salt thereof, the method comprising reacting an aniline of formula (A1) with an aldehyde of formula (A2) in the presence of boron trifluoride-tetrahydrofuran complex or boron trifluoride-diethyl ether complex, and subsequently reacting the resulting compound with an α-keto acid of formula (A3).
2) The method according to 1), wherein the aniline of formula (A1) is 4-(trifluoromethoxy)aniline, the aldehyde of formula (A2) is benzaldehyde, and the α-keto acid of formula (A3) is pyruvic acid.
3) A method for producing a quinolinecarboxamide derivative of formula (A6) or a salt thereof, the method comprising reacting a quinolinecarboxylic acid derivative of formula (A4) or a salt thereof produced by a method according to 1) or 2) with 5-(furan-2-yl)-1,3,4-oxadiazol-2-amine of formula (A5).

The present inventors also found that in the case of producing an isatin derivative from an aniline, an isatin derivative of formula (B4) can be produced at high yields by using a 2-(alkoxyimino)acetic acid, as shown below, and by extension, a quinolinecarboxamide derivative of formula (B8) or a salt thereof can be efficiently produced: 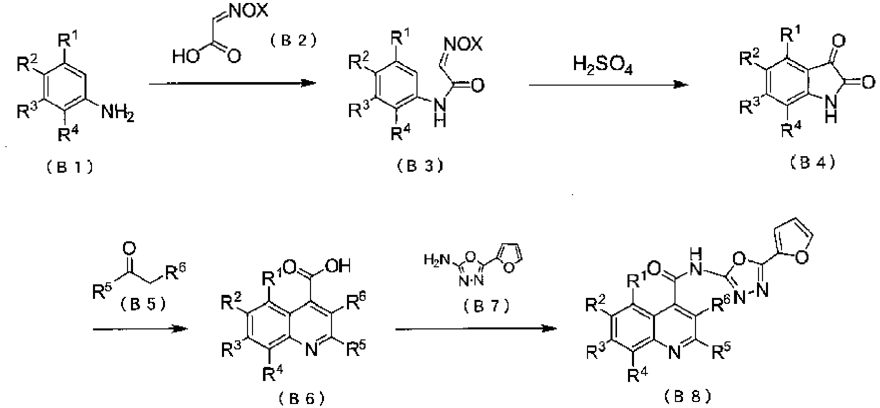 wherein R¹, R², R³ and R⁴ are the same or different and each represent a hydrogen atom, a halogen atom, a lower alkyl group, a nitro group, a hydroxy group, a cyano group, an optionally substituted phenyl group, an optionally substituted 5- or 6-membered heterocyclic group, a lower alkoxy group, a halo-lower alkoxy group, a lower alkoxy-lower alkoxy group, a lower alkyl-carbonyl group, a lower alkoxy-carbonyl group, a benzyloxy group, a trityloxy group, a t-butyldimethylsilyloxy group, a di-lower alkyl-amino group, a t-butoxycarbonylamino group, a benzyloxycarbonylamino group, a 2-nitrobenzenesulfonylamino group or a lower alkyl-thio group, R⁵ represents a hydrogen atom, a lower alkyl group, a cyclo-lower alkyl group, an optionally substituted phenyl group, an optionally substituted 5- or 6-membered heterocyclic group, a naphthyl group, a 1,3-benzodioxolyl group, a styryl group, or -CR^{5a}OR^{5b}CH₂OR^{5c}, wherein R^{5a} represents a methyl group or a t-butyl group, and R^{5b} and R^{5c} are the same or different and each represent a hydrogen atom, a lower alkyl group, a lower alkanoyl group, a t-butyldimethylsilyl group, a 2-(trimethylsilyl)ethoxymethyl group, a benzyloxymethyl group, a phenyl group or a benzyl group optionally having a substituent on the ring, a lower alkenyl group or a lower alkoxy-methyl group, R⁶ represents a hydrogen atom, a lower alkyl group, a hydroxy group, an aryl group or a halogen atom, and X represents a lower alkyl group.

Specifically, a second aspect of the invention relates to the following 1) to 6).
1) A method for producing an isatin derivative of formula (B4) or a salt thereof, the method comprising reacting an aniline of formula (B1) with a 2-(alkoxyimino)acetic acid of formula (B2) to prepare a 2-(alkoxyimino)-acetamide of formula (B3), and heating this compound in concentrated sulfuric acid.
2) The method according to 1), wherein the aniline of formula (B1) is 4-(trifluoromethoxy)aniline.
3) A method for producing a quinolinecarboxylic acid derivative of formula (B6) or a salt thereof, the method comprising reacting an isatin derivative of formula (B4) or a salt thereof produced by a method according to 1) or 2) with a ketone of formula (B5) in the presence of a base.
4) The method according to 3), wherein the ketone of formula (B5) is acetophenone.
5) The method according to 3) or 4), wherein the base is sodium hydroxide.
6) A method for producing a quinolinecarboxamide derivative of formula (B8) or a salt thereof, the method comprising reacting a quinolinecarboxylic acid derivative of formula (B6) or a salt thereof produced by a method according to any of 3) to 5) with 5-(furan-2-yl)-1,3,4-oxadiazol-2-amine of formula (B7).

The present inventors also found that a 1,3,4-oxa(thia)diazol-2-amine derivative of formula (C4) can be efficiently produced by reacting an aldehyde of formula (C1) with semicarbazide or thiosemicarbazide of formula (C2) or a salt thereof, and cyclizing the resulting semicarbazone or thiosemicarbazone of formula (C3), without isolation, using chloramine T: 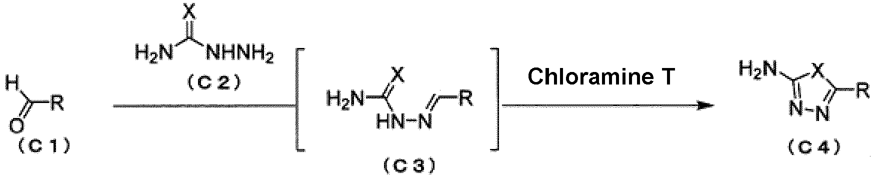 wherein R represents a hydrogen atom, a lower alkyl group, a lower alkenyl group, a cyclo-lower alkyl group, an optionally substituted phenyl group, an optionally substituted 5- or 6-membered heterocyclic group, a benzyl group, a naphthyl group or a styryl group, and X represents an oxygen atom or a sulfur atom.

Specifically, a third aspect of the invention relates to the following 1) to 2).
1) A method for producing a 1,3,4-oxa(thia)diazol-2-amine derivative of formula (C4) or a salt thereof, the method comprising reacting an aldehyde of formula (C1) with semicarbazide or thiosemicarbazide of formula (C2) or a salt thereof, and cyclizing the resulting semicarbazone or thiosemicarbazone of formula (C3), without isolation, using chloramine T.
2) The method according to 1), wherein the aldehyde of formula (C1) is furfural, and the semicarbazide or the thiosemicarbazide of formula (C2) or the salt thereof is semicarbazide or a salt thereof.

### Effects of the Invention

According to the first aspect of the invention, a quinolinecarboxylic acid derivative of formula (A4) or a salt thereof can be produced at high yields, and by extension, a quinolinecarboxamide derivative of formula (A6) or a salt thereof which is an inhibitor of STAT3 can be industrially advantageously produced.

According to the second aspect of the invention, an isatin derivative of formula (B4) or a salt thereof can be produced at high yields, and by extension, a quinolinecarboxamide derivative of formula (B8) or a salt thereof which is an inhibitor of STAT3 can be industrially advantageously produced.

According to the third aspect of the invention, 1,3,4-oxa(thia)diazol-2-amine or a salt thereof which may be used in the production of STAT3 inhibitors including the quinolinecarboxamide derivative of formula (A6) or (B8) can be industrially advantageously produced.

### Detailed Description of the Invention

The term "lower" as used herein means that the number of carbon atoms in the hydrocarbon moiety of a group with this term is from 1 to 9 for a chain hydrocarbon moiety and from 3 to 7 for a cyclic hydrocarbon moiety and means that the chain hydrocarbon moiety may be linear or branched, unless otherwise specified. Herein, the number of carbon atoms (from x to y carbon atoms) in a hydrocarbon moiety is abbreviated to "C_{x-y}".

The term "optionally substituted" or "optionally having a substituent" means that a hydrogen atom of the group of interest may be replaced with another group. The number of the substituent can be one or more. When two or more substituents are present, the substituents may be the same or different.

In formula (A1) to (A6) and (B1) to (B8) according to the present invention, examples of the halogen atom represented by R¹, R², R³, R⁴ and R⁶ include fluorine, chlorine, bromine and iodine. Fluorine, chlorine or bromine is preferred.

The lower alkyl group of each of R¹, R², R³, R⁴, R⁵ and R⁶ is preferably a C₁₋₉ alkyl group such as a methyl group, an ethyl group, a propyl group, an isopropyl group, a butyl group, an isobutyl group, a tert-butyl group, a pentyl group, a hexyl group, a heptyl group, an octyl group, or a nonyl group, more preferably a methyl group, an ethyl group, a propyl group, a tert-butyl group, or a nonyl group.

The lower alkoxy group of each of R¹, R², R³ and R⁴ is preferably a C₁₋₄ alkoxy group such as a methoxy group, an ethoxy group, a propoxy group, an isopropoxy group, or a butoxy group, more preferably a methoxy group.

The halo-lower alkoxy group of each of R¹, R², R³ and R⁴ is preferably a halo-C₁₋₄ alkoxy group. Examples thereof include a difluoromethoxy group, a trifluoromethoxy group, and a 2,2,2-trifluoroethoxy group. A trifluoromethoxy group is preferred.

The lower alkoxy-lower alkoxy group of each of R¹, R², R³ and R⁴ is preferably a C₁₋₄ alkoxy-C₁₋₄ alkoxy group. Examples thereof include a methoxymethoxy group, an ethoxymethoxy group, a methoxyethoxy group, and an ethoxyethoxy group. A methoxymethoxy group or a methoxyethoxy group is preferred.

The lower alkyl-carbonyl group of each of R¹, R², R³ and R⁴ is preferably a C₁₋₄ alkylcarbonyl group. Examples thereof include a methylcarbonyl group, an ethylcarbonyl group, a n-propylcarbonyl group, and a tert-butylcarbonyl group. A methylcarbonyl group is preferred.

The lower alkoxy-carbonyl group of each of R¹, R², R³ and R⁴ is preferably a C₁₋₄ alkoxycarbonyl group. Examples thereof include a methoxycarbonyl group, an ethoxycarbonyl group, a n-propoxycarbonyl group, and a tert-butoxycarbonyl group. A methoxycarbonyl group is preferred.

The di-lower alkyl-amino group of each of R¹, R², R³ and R⁴ is preferably a di-C₁₋₄ alkylamino group. Examples thereof include a dimethylamino group, a diethylamino group, a di-n-propylamino group, and a diisopropylamino group. A dimethylamino group is preferred.

The lower alkyl-thio group of each of R¹, R², R³ and R⁴ is preferably a C₁₋₄ alkylthio group. Examples thereof include a methylthio group, an ethylthio group, a n-propylthio group, and an isopropylthio group. A methylthio group is preferred.

In the optionally substituted phenyl group of each of R¹, R², R³, R⁴ and R⁵, examples of the group which can be a substituent on the phenyl group include halogen atoms (e.g., a chlorine atom and a bromine atom), C₁₋₄ alkyl groups (e.g., a methyl group, an ethyl group, a propyl group, an isopropyl group, a butyl group, an isobutyl group, and a tert-butyl group), C₂₋₇ alkenyl groups (e.g., a vinyl group, a propenyl group, a 2-methyl-1-propenyl group, and a 1-methyl-1-propenyl group), C₁₋₄ alkoxy groups (e.g., a methoxy group, an ethoxy group, a propoxy group, an isopropoxy group, and a butoxy group), halo-C₁₋₄ alkoxy groups (e.g., a trifluoromethoxy group), a hydroxy group, a nitro group, a cyano group, C₁₋₄ alkylcarbonyl groups (e.g., a methylcarbonyl group), C₁₋₄ alkoxycarbonyl groups (e.g., a methoxycarbonyl group), di-C₁₋₄ alkylamino groups (e.g., a dimethylamino group), a t-butoxycarbonylamino group, a benzyloxycarbonylamino group, and a 2-nitrobenzenesulfonylamino group.

In the optionally substituted 5- or 6-membered heterocyclic group of each of R¹, R², R³, R⁴ and R⁵, examples of the 5- or 6-membered heterocyclic group include a pyrrolyl group, a pyrazolyl group, a furyl group, a thienyl group, a pyridyl group, an imidazolyl group, a triazolyl group, a tetrazolyl group, a triazinyl group, a pyridazinyl group, a pyrimidinyl group, a pyrazinyl group, an isoxazolyl group, a thiazolyl group, an isothiazolyl group, a thiadiazolyl group, an oxazolyl group, and an oxadiazolyl group. A pyridyl group, a furyl group, a thienyl group, or a pyrrolyl group is preferred.

Examples of the group which can be a substituent on the heterocyclic group include halogen atoms (e.g., a chlorine atom and a bromine atom), C₁₋₄ alkyl groups (e.g., a methyl group, an ethyl group, a propyl group, an isopropyl group, a butyl group, an isobutyl group, and a tert-butyl group), C₂₋₇ alkenyl groups (e.g., a vinyl group, a propenyl group, a 2-methyl-1-propenyl group, and a 1-methyl-1-propenyl group), C₁₋₄ alkoxy groups (e.g., a methoxy group, an ethoxy group, a propoxy group, an isopropoxy group, and a butoxy group), halo-C₁₋₄ alkoxy groups (e.g., a trifluoromethoxy group), a hydroxy group, a nitro group, a cyano group, C₁₋₄ alkylcarbonyl groups (e.g., a methylcarbonyl group), C₁₋₄ alkoxycarbonyl groups (e.g., a methoxycarbonyl group), di-C₁₋₄ alkylamino groups (e.g., a dimethylamino group), a t-butoxycarbonylamino group, a benzyloxycarbonylamino group, and a 2-nitrobenzenesulfonylamino group.

The cyclo-lower alkyl group of R⁵ is preferably a cyclo-C₃₋₇ alkyl group. Examples thereof include a cyclopropyl group, a cyclobutyl group, a cyclopentyl group, and a cyclohexyl group. A cyclohexyl group is preferred.

In -CR^{5a}OR^{5b}CH₂OR^{5c} of R⁵, examples of the lower alkyl group of each of R^{5b} and R^{5c} include C₁₋₄ alkyl groups (e.g., a methyl group, an ethyl group, a propyl group, an isopropyl group, a butyl group, an isobutyl group, and a tert-butyl group). Examples of the lower alkanoyl group include C₁₋₇ alkanoyl groups (e.g., an acetyl group, a propionyl group, a butyryl group, and a valeryl group). In the phenyl group or the benzyl group optionally having a substituent on the ring, examples of the substituent include C₁₋₄ alkoxy groups (e.g., a methoxy group, an ethoxy group, a propoxy group, an isopropoxy group, and a butoxy group) and halogen atoms (e.g., a chlorine atom and a bromine atom). Examples of the lower alkenyl group include C₂₋₇ alkenyl groups (e.g., a vinyl group, a propenyl group, a 2-methyl-1-propenyl group, and a 1-methyl-1-propenyl group). Examples of the lower alkoxy-methyl group include C₁₋₄ alkoxymethyl groups (e.g., a methoxymethyl group, an ethoxymethyl group, a propoxymethyl group, an isopropoxymethyl group, and a butoxymethyl group).

Examples of the aryl group of R⁶ include C₆₋₁₄ aryl groups such as a phenyl group, a naphthyl group, and an indenyl group. A phenyl group is preferred.

As for R¹ to R⁴, preferably, R¹ is a hydrogen atom, R² is a hydrogen atom, a halogen atom, a lower alkyl group, a nitro group, a hydroxy group, a cyano group, an optionally substituted phenyl group, an optionally substituted 5- or 6-membered heterocyclic group, a lower alkoxy group, a halo-lower alkoxy group, a lower alkoxy-lower alkoxy group, a lower alkyl-carbonyl group, a lower alkoxy-carbonyl group, a benzyloxy group, a trityloxy group, a t-butyldimethylsilyloxy group, a di-lower alkyl-amino group, a t-butoxycarbonylamino group, a benzyloxycarbonylamino group, a 2-nitrobenzenesulfonylamino group, or a lower alkyl-thio group, and R³ and R⁴ are the same or different and each are a hydrogen atom, a halogen atom, a lower alkyl group or a nitro group.

As for R¹ to R⁶, particularly preferably, each of R¹, R³ and R⁴ is a hydrogen atom, R² is a halo-C₁₋₄ alkoxy group (preferably a trifluoromethoxy group), R⁵ is an optionally substituted phenyl group (preferably a phenyl group), and R⁶ is a hydrogen atom.

Examples of the salt of the quinolinecarboxylic acid derivative of formula (A4) (hereinafter, referred to as the quinolinecarboxylic acid derivative (A4)), the quinolinecarboxamide derivative of formula (A6) (hereinafter, referred to as the quinolinecarboxamide derivative (A6)), the isatin derivative of formula (B4) (hereinafter, referred to as the isatin derivative (B4)), the quinolinecarboxylic acid derivative of formula (B6) (hereinafter, referred to as the quinolinecarboxylic acid derivative (B6)), or the quinolinecarboxamide derivative of formula (B8) (hereinafter, referred to as the quinolinecarboxamide derivative (B8)) include pharmacologically acceptable acid-addition salts, metal salts, ammonium salts, organic amine-addition salts, and amino acid-addition salts. Examples of the pharmacologically acceptable acid-addition salt include salts of inorganic acids such as hydrochloric acid, hydrobromic acid, sulfuric acid, nitric acid, phosphoric acid, and boric acid, and salts of organic acids including carboxylic acids such as formic acid, acetic acid, propionic acid, fumaric acid, malonic acid, succinic acid, maleic acid, tartaric acid, citric acid, and benzoic acid, sulfonic acids such as methanesulfonic acid and p-toluenesulfonic acid, and amino acids such as glutamic acid and aspartic acid. Examples of the pharmacologically acceptable metal salt include salts of alkali metals such as lithium, sodium, and potassium, salts of alkaline earth metals such as magnesium and calcium, and salts of metals such as aluminum and zinc. Examples of the pharmacologically acceptable ammonium salt include salts of ammonium and tetramethylammonium. Examples of the pharmacologically acceptable organic amine-addition salt include salts of triethylamine, piperidine, morpholine, and toluidine. Examples of the pharmacologically acceptable amino acid-addition salt include addition salts of lysine, glycine, and phenylalanine.

### <First aspect of invention>

Hereinafter, the methods for producing the quinolinecarboxylic acid derivative (A4) or the salt thereof, and the quinolinecarboxamide derivative (A6) or the salt thereof according to the present invention will be described in detail. All starting materials and produced compounds may be in the form of salts. A compound produced through each reaction can be converted to a salt by a routine method.

A1. Production of quinolinecarboxylic acid derivative (A4) or salt thereof

This reaction is performed by reacting an aniline of formula (A1) (hereinafter, referred to as an aniline (A1)) with an aldehyde of formula (A2) (hereinafter, referred to as an aldehyde (A2)) in the presence of boron trifluoride-tetrahydrofuran complex or boron trifluoride-diethyl ether complex, and subsequently reacting the resulting compound with an α-keto acid of formula (A3) (hereinafter, referred to as an α-keto acid (A3)).

The reaction of the aniline (A1) with the aldehyde (A2) is performed in an appropriate solvent in the presence of boron trifluoride-tetrahydrofuran complex or boron trifluoride-diethyl ether complex.

The amount of the aniline (A1) used can usually be from 1.0 to 2.2 times by mole, preferably from 1.7 to 1.9 times by mole, the amount of the α-keto acid (A3).

The amount of aldehyde (A2) used can usually be from 1.0 to 2.4 times by mole, preferably from 1.9 to 2.1 times by mole, the amount of the α-keto acid (A3).

The amount of the boron trifluoride-tetrahydrofuran complex or the boron trifluoride-diethyl ether complex for use in this reaction is from 0.1 to 1.0 times by mole, preferably from 0.4 to 1.0 times by mole the amount of the α-keto acid (A3).

The reaction is performed in an appropriate solvent. The solvent used is not particularly limited as long as the solvent does not participate in the reaction. Examples thereof include nitrile solvents such as acetonitrile, isobutyronitrile, propionitrile, and methoxyacetonitrile, ether solvents such as diethyl ether, tetrahydrofuran (THF), 2-methyltetrahydrofuran, 1,3- or 1,4-dioxane, t-butyl methyl ether (MTBE), cyclopentyl methyl ether (CPME), 1,2-dimethoxyethane (DME), and diethylene glycol dimethyl ether, aprotic solvents such as N,N-dimethylformamide (DMF) and dimethyl sulfoxide (DMSO), halogenated hydrocarbons such as dichloromethane, chloroform, and 1,2-dichloroethane, and aromatic hydrocarbons such as toluene, o-xylene, m-xylene, and p-xylene. Among them, a nitrile solvent, an ether solvent, an aprotic solvent, or a halogenated hydrocarbon is preferred, and acetonitrile is more preferred. These solvents may be used alone or in combination, and the amount of the solvent used is not particularly limited.

The reaction temperature can usually be in the range of -78°C to the boiling point of the solvent used and is preferably from 60 to 70°C.

The reaction time is usually preferably from 0 to 24 hours, more preferably from 10 minutes to 1 hour.

Subsequently, the reaction product obtained through the reaction is reacted with an α-keto acid (A3) to obtain a quinolinecarboxylic acid derivative (A4) or a salt thereof.

For the reaction, for example, the α-keto acid (A3) dissolved or suspended in the solvent described above is added to the reaction solution from the reaction described above over usually preferably from 0 to 24 hours, more preferably from 0 to 12 hours.

The reaction is preferably performed with stirring at a reaction temperature usually in the range of -78°C to the boiling point of the solvent used, preferably from 60 to 70°C, for a time usually from 5 minutes to 45 hours, preferably from 12 to 24 hours.

The aniline (A1), the aldehyde (A2) and the α-keto acid (A3) may be obtained as commercially available products or can be obtained by a method described in a literature, etc. or a method equivalent thereto.

After completion of the reaction, the quinolinecarboxylic acid derivative (A4) or the salt thereof can be separated by adding an organic solvent such as toluene, diethyl ether, diisopropyl ether (IPE), t-butyl methyl ether (MTBE), or tetrahydrofuran (THF) to the reaction solution, appropriately washing the mixture with water, subjecting the organic layer to extraction operation using a basic aqueous solution of sodium bicarbonate, sodium carbonate, sodium hydroxide, or the like, performing crystallization by adding an acidic aqueous solution of hydrochloric acid or the like to the obtained aqueous layer, and collecting the resulting crystals by filtration; by adding water or aqueous sodium chloride solution to the reaction solution, performing extraction operation using an organic solvent such as toluene, diethyl ether, diisopropyl ether (IPE), t-butyl methyl ether (MTBE), or tetrahydrofuran (THF), and drying the obtained organic layer according to a routine method; or by collecting a solid precipitated in the reaction solution by filtration. The quinolinecarboxylic acid derivative (A4) or the salt thereof may be subjected to purification operation by silica gel column chromatography or by suspending, washing or recrystallization by using an organic solvent such as acetone, methyl ethyl ketone (MEK), methyl isobutyl ketone (MIBK), ethyl acetate, butyl acetate, methanol, ethanol, 1- or 2-propanol, dichloromethane, chloroform, acetonitrile, diethyl ether, diisopropyl ether (IPE), t-butyl methyl ether (MTBE), tetrahydrofuran (THF), 1,3- or 1,4-dioxane, heptane, or hexane alone or in combination.

It has previously been reported that such a quinolinecarboxylic acid derivative is produced by reacting an aniline, an aldehyde and an α-keto acid in the presence or absence of an acid such as sulfamic acid (Non Patent Literatures A1 and A2 described above). However, there has been a problem in such a reaction in that the yield decreases depending on the type of a substituent on the aniline, particularly, when R² has an electron-withdrawing substituent. By contrast, the method of the present invention using boron trifluoride-tetrahydrofuran complex or boron trifluoride-diethyl ether complex enables production of a quinolinecarboxylic acid derivative (A4) or a salt thereof at high yields (see Comparative Examples described later).

A2. Production of quinolinecarboxamide derivative (A6) or salt thereof

The quinolinecarboxamide derivative (A6) or the salt thereof can be produced in accordance with a method described in Patent Literature 1 described above.

Specifically, the compound can be obtained by reacting the quinolinecarboxylic acid derivative (A4) or the salt thereof obtained as described above with 5-(furan-2-yl)-1,3,4-oxadiazol-2-amine (A5) at a temperature from -78°C to the boiling point of the solvent used for a time from 5 minutes to 48 hours in an appropriate inert solvent, for example, a halogenated hydrocarbon such as chloroform or dichloromethane, an aromatic hydrocarbon such as benzene or toluene, an ether solvent such as diethyl ether, tetrahydrofuran (THF), or 1,4-dioxane, an aprotic polar solvent such as N,N-dimethylformamide (DMF), N-methylpyrrolidone (NMP), or dimethyl sulfoxide (DMSO), a basic solvent such as pyridine or quinoline, or a mixed solvent thereof in the presence of a base and a condensing agent.

Examples of the base include organic bases such as triethylamine, N,N-diisopropylethylamine (DIPEA), N-methylmorpholine (NMM), and pyridine, inorganic bases such as potassium carbonate, potassium bicarbonate, tripotassium phosphate, sodium hydroxide, and sodium hydride, and metal alkoxides such as sodium methoxide and potassium tert-butoxide.

Examples of the condensing agent that can be used include carbodiimide condensing agents such as DCC and WSCI, phosphonium condensing agents such as BOP, aminium/uronium condensing agents such as HATU and HBTU, triazine condensing agents such as DMT-MM, imidazole condensing agents such as CDI, phosphinic acid chloride condensing agents such as DPP-Cl, and phosphonic anhydride condensing agents such as T3P.

After completion of the reaction, the quinolinecarboxamide derivative (A6) or the salt thereof can be obtained by washing the reaction solution with aqueous sodium chloride solution, water, or the like, followed by drying according to a routine method; or by adding aqueous sodium chloride solution or water to the reaction solution, and collecting the resulting solid by filtration. The quinolinecarboxamide derivative (A6) or the salt thereof may be subjected to purification operation by silica gel column chromatography or by suspending, washing or recrystallization by using an organic solvent such as acetone, methyl ethyl ketone (MEK), methyl isobutyl ketone (MIBK), ethyl acetate, butyl acetate, methanol, ethanol, 1- or 2-propanol, dichloromethane, chloroform, acetonitrile, diethyl ether, diisopropyl ether (IPE), t-butyl methyl ether (MTBE), tetrahydrofuran (THF), 1,3- or 1,4-dioxane, heptane, or hexane alone or in combination.

### <Second aspect of invention>

Hereinafter, the methods for producing the isatin derivative (B4) or the salt thereof, the quinolinecarboxylic acid derivative (B6) or the salt thereof, and the quinolinecarboxamide derivative (B8) or the salt thereof according to the present invention will be described in detail. All starting materials and produced compounds may be in the form of salts. A compound produced through each reaction can be converted to a salt by a routine method.

B1. Production of isatin derivative (B4) or salt thereof
This reaction is performed by reacting an aniline of formula (B1) (hereinafter, referred to as an aniline (B1)) with a 2-(alkoxyimino)acetic acid of formula (B2) (hereinafter, referred to as a 2-(alkoxyimino)acetic acid (B2)) to prepare a 2-(alkoxyimino)-acetamide of formula (B3) (hereinafter, referred to as a 2-(alkoxyimino)-acetamide (B3)), and heating this compound in concentrated sulfuric acid.
The reaction of the aniline (B1) with the 2-(alkoxyimino)acetic acid (B2) is performed in an appropriate solvent using a condensing agent.

The condensing agent can be a condensing agent for use in amide bond formation. Examples thereof include carbodiimide condensing agents such as DCC and WSCI, phosphonium condensing agents such as BOP, aminium/uronium condensing agents such as HATU and HBTU, triazine condensing agents such as DMT-MM, imidazole condensing agents such as CDI, phosphinic acid chloride condensing agents such as DPP-Cl, and phosphonic anhydride condensing agents such as T3P.

The amount of condensing agent used is from 1.05 to 1.30 times by mole, preferably 1.15 times by mole, the amount of the aniline (B1).

The reaction is performed in an appropriate solvent. The solvent used is not particularly limited as long as the solvent does not participate in the reaction. Examples thereof include nitrile solvents such as acetonitrile, isobutyronitrile, propionitrile, and methoxyacetonitrile, ether solvents such as diethyl ether, tetrahydrofuran (THF), 2-methyltetrahydrofuran, 1,3- or 1,4-dioxane, t-butyl methyl ether (MTBE), cyclopentyl methyl ether (CPME), 1,2-dimethoxyethane (DME), and diethylene glycol dimethyl ether, ester solvents such as ethyl acetate and butyl acetate, aprotic solvents such as N,N-dimethylformamide (DMF) and dimethyl sulfoxide (DMSO), halogenated hydrocarbons such as dichloromethane, chloroform, and 1,2-dichloroethane, and aromatic hydrocarbons such as toluene, o-xylene, m-xylene, and p-xylene. Among them, a nitrile solvent, an ether solvent, an ester solvent, a halogenated hydrocarbon, or an aprotic solvent is preferred, and acetonitrile is more preferred. These solvents may be used alone or in combination, and the amount of the solvent used is not particularly limited.

The reaction can usually be performed at a temperature in the range of -78°C to the boiling point of the solvent used, preferably from 0°C to 40°C, under an inert gas. The reaction time is usually preferably from 5 minutes to 24 hours, more preferably from 10 minutes to 6 hours.

Subsequently, the reaction product obtained through the reaction is reacted with a 2-(alkoxyimino)acetic acid (B2) to obtain a 2-(alkoxyimino)-acetamide (B3).

The amount of 2-(alkoxyimino)acetic acid (B2) used may usually be from 1.10 to 1.35 times by mole, preferably 1.25 times by mole the amount of the aniline (B1) .

The reaction can usually be performed at a temperature in the range of -78°C to the boiling point of the solvent used, preferably from 15°C to the boiling point of the solvent used, under an inert gas. The reaction time is usually preferably from 5 minutes to 24 hours, more preferably from 30 minutes to 8 hours.

Examples of the 2-(alkoxyimino)acetic acid (B2) for use in this reaction preferably include 2-(methoxyimino)acetic acid. 2-(Methoxyimino)acetic acid can be synthesized by a routine method from glyoxylic acid and methoxylamine hydrochloride which is commonly used as an oximation reagent, as shown in Reference Examples mentioned later.

The 2-(alkoxyimino)-acetamide (B3) obtained through the reaction is heated in concentrated sulfuric acid to obtain an isatin derivative (B4) or a salt thereof.

This reaction is preferably performed with stirring at a reaction temperature usually in the range of 15 to 290°C, preferably from 60 to 80°C, for a time usually from 5 minutes to 42 hours, preferably from 3 to 24 hours.

The aniline (B1) and the 2-(alkoxyimino)acetic acid (B2) may be obtained as commercially available products or can be obtained by a method described in a literature, etc. or a method equivalent thereto.

After completion of the reaction, the reaction product can be separated by a separation approach known in the art such as filtration, and appropriately washed with water to obtain an isatin derivative (B4) or a salt thereof.

In this way, the method enables the production of the isatin derivative (B4) or the salt thereof at high yields without using explosive hydroxylamine and without generating by-products derived from the aniline (B1) even used with a high concentration.

B2. Production of quinolinecarboxylic acid derivative (B6) or salt thereof

The quinolinecarboxylic acid derivative (B6) or the salt thereof can be produced by use of a Pfitzinger quinoline synthesis method from a corresponding isatin derivative.

Specifically, the compound can be produced by reacting the isatin derivative of formula (B4) or the salt thereof obtained as described above with a ketone of formula (B5) (referred to as ketone (B5)) under basic conditions.

The reaction is desirably performed in an alcohol (e.g., methanol, ethanol, and 1- or 2-propanol) and/or water solvent in the presence of an alkali such as sodium hydroxide or potassium hydroxide.

The amount of ketone (B5) is usually from 1.00 to 5.00 times by mole, preferably from 1.05 to 3.15 times by mole the amount of the isatin derivative (B4) or the salt thereof.

The reaction is preferably performed with stirring at a reaction temperature usually in the range of -78°C to the boiling point of the solvent used, preferably from 60°C to the boiling point of the solvent used, for a time usually from 5 minutes to 58 hours, preferably from 6 to 30 hours.

After completion of the reaction, the quinolinecarboxylic acid derivative (B6) or the salt thereof can be separated by appropriately distilling off the solvent under reduced pressure from the reaction solution, adding an organic solvent such as toluene, diethyl ether, diisopropyl ether (IPE), t-butyl methyl ether (MTBE), or tetrahydrofuran (THF) to the residue, performing extraction operation using water or a basic aqueous solution of sodium bicarbonate, sodium carbonate, sodium hydroxide, or the like, performing crystallization by adding an acidic aqueous solution of hydrochloric acid or the like to the obtained aqueous layer, and collecting the resulting crystals by filtration; by adding an acidic aqueous solution of hydrochloric acid or the like to the residue, performing extraction operation using an organic solvent such as toluene, diethyl ether, diisopropyl ether (IPE), t-butyl methyl ether (MTBE), tetrahydrofuran (THF), ethyl acetate, dichloromethane, or chloroform, and drying the obtained organic layer according to a routine method; or by collecting a solid precipitated in the reaction solution by filtration. The quinolinecarboxylic acid derivative (B6) or the salt thereof may be purification operation by silica gel column chromatography or by suspending, washing or recrystallization by using an organic solvent such as acetone, methyl ethyl ketone (MEK), methyl isobutyl ketone (MIBK), ethyl acetate, butyl acetate, methanol, ethanol, 1- or 2-propanol, dichloromethane, chloroform, acetonitrile, diethyl ether, diisopropyl ether (IPE), t-butyl methyl ether (MTBE), tetrahydrofuran (THF), 1,3- or 1,4-dioxane, heptane, or hexane alone or in combination.

B3. Production of quinolinecarboxamide derivative (B8) or salt thereof

The quinolinecarboxamide derivative (B8) or the salt thereof can be produced in accordance with a method described in Patent Literature 1 described above.

Specifically, the compound can be obtained by reacting the quinolinecarboxylic acid derivative (B6) or the salt thereof obtained as described above with 5-(furan-2-yl)-1,3,4-oxadiazol-2-amine (B7) at a temperature from -78°C to the boiling point of the solvent used for a time from 5 minutes to 48 hours in an appropriate inert solvent, for example, a halogenated hydrocarbon such as chloroform or dichloromethane, an aromatic hydrocarbon such as benzene or toluene, an ether solvent such as diethyl ether, tetrahydrofuran (THF), or 1,4-dioxane, an aprotic polar solvent such as N,N-dimethylformamide (DMF), N-methylpyrrolidone (NMP), or dimethyl sulfoxide (DMSO), a basic solvent such as pyridine or quinoline, or a mixed solvent thereof in the presence of a base and a condensing agent.

Examples of the base include organic bases such as triethylamine, N,N-diisopropylethylamine (DIPEA), N-methylmorpholine (NMM), and pyridine, inorganic bases such as potassium carbonate, potassium bicarbonate, tripotassium phosphate, sodium hydroxide, and sodium hydride, and metal alkoxides such as sodium methoxide and potassium tert-butoxide.

Examples of the condensing agent that can be used include carbodiimide condensing agents such as DCC and WSCI, phosphonium condensing agents such as BOP, aminium/uronium condensing agents such as HATU and HBTU, triazine condensing agents such as DMT-MM, imidazole condensing agents such as CDI, phosphinic acid chloride condensing agents such as DPP-Cl, and phosphonic anhydride condensing agents such as T3P.

After completion of the reaction, the quinolinecarboxamide derivative (B8) or the salt thereof can be obtained by washing the reaction solution with aqueous sodium chloride solution, water, or the like, followed by drying according to a routine method; or by adding aqueous sodium chloride solution or water to the reaction solution, and collecting the resulting solid by filtration. The quinolinecarboxamide derivative (B8) or the salt thereof may be subjected to purification operation by silica gel column chromatography or by suspending, washing or recrystallization by using an organic solvent such as acetone, methyl ethyl ketone (MEK), methyl isobutyl ketone (MIBK), ethyl acetate, butyl acetate, methanol, ethanol, 1- or 2-propanol, dichloromethane, chloroform, acetonitrile, diethyl ether, diisopropyl ether (IPE), t-butyl methyl ether (MTBE), tetrahydrofuran (THF), 1,3- or 1,4-dioxane, heptane, or hexane alone or in combination.

### <Third aspect of invention>

In formula (C1) to (C4) according to the present invention, the lower alkyl group of R is preferably a C₁₋₉ alkyl group such as a methyl group, an ethyl group, a propyl group, an isopropyl group, a butyl group, an isobutyl group, a tert-butyl group, a pentyl group, a hexyl group, a heptyl group, an octyl group, or a nonyl group, more preferably a methyl group, an ethyl group, a propyl group, a tert-butyl group, or a nonyl group.

The lower alkenyl group of R is preferably, for example, a vinyl group, a propenyl group, a 2-methyl-1-propenyl group, or a 1-methyl-1-propenyl group, more preferably a vinyl group or a 2-methyl-1-propenyl group.

The cyclo-lower alkyl group of R is preferably a cyclo-C₃₋₇ alkyl group. Examples thereof include a cyclopropyl group, a cyclobutyl group, a cyclopentyl group, and a cyclohexyl group. A cyclohexyl group is preferred.

In the optionally substituted phenyl group of R, examples of the group which can be a substituent on the phenyl group include halogen atoms (e.g., a chlorine atom and a bromine atom), C₁₋₄ alkyl groups (e.g., a methyl group, an ethyl group, a propyl group, an isopropyl group, a butyl group, an isobutyl group, and a tert-butyl group), C₂₋₇ alkenyl groups (e.g., a vinyl group, a propenyl group, a 2-methyl-1-propenyl group, and a 1-methyl-1-propenyl group), C₁₋₄ alkoxy groups (e.g., a methoxy group, an ethoxy group, a propoxy group, an isopropoxy group, and a butoxy group), a hydroxy group, a nitro group, a cyano group, C₁₋₄ alkylcarbonyl groups (e.g., a methylcarbonyl group), C₁₋₄ alkoxycarbonyl groups (e.g., a methoxycarbonyl group), an amino group, di-C₁₋₄ alkylamino groups (e.g., a dimethylamino group), a t-butoxycarbonylamino group, a benzyloxycarbonylamino group, and a 2-nitrobenzenesulfonylamino group as well as divalent groups which bond two carbon atoms on a ring, such as an oxyethylene group and an oxyethyleneoxy group.

In the optionally substituted 5- or 6-membered heterocyclic group of R, examples of the 5- or 6-membered heterocyclic group include a pyrrolyl group, a pyrazolyl group, a furyl group, a thienyl group, a pyridyl group, an imidazolyl group, a triazolyl group, a tetrazolyl group, a triazinyl group, a pyridazinyl group, a pyrimidinyl group, a pyrazinyl group, an isoxazolyl group, a thiazolyl group, an isothiazolyl group, a thiadiazolyl group, an oxazolyl group, and an oxadiazolyl group. A pyridyl group, a furyl group, or a thienyl group is preferred, and a 2-furyl group or a 3-furyl group is more preferred.

Examples of the group which can be a substituent on the heterocyclic group include halogen atoms (e.g., a chlorine atom and a bromine atom), C₁₋₄ alkyl groups (e.g., a methyl group, an ethyl group, a propyl group, an isopropyl group, a butyl group, an isobutyl group, and a tert-butyl group), C₂₋₇ alkenyl groups (e.g., a vinyl group, a propenyl group, a 2-methyl-1-propenyl group, and a 1-methyl-1-propenyl group), C₁₋₄ alkoxy groups (e.g., a methoxy group, an ethoxy group, a propoxy group, an isopropoxy group, and a butoxy group), a hydroxy group, a nitro group, a cyano group, C₁₋₄ alkylcarbonyl groups (e.g., a methylcarbonyl group), C₁₋₄ alkoxycarbonyl groups (e.g., a methoxycarbonyl group), an amino group, di-C₁₋₄ alkylamino groups (e.g., a dimethylamino group), a t-butoxycarbonylamino group, a benzyloxycarbonylamino group, and a 2-nitrobenzenesulfonylamino group.

Examples of the naphthyl group of R specifically include a 1-naphthyl group and a 2-naphthyl group.

In the chemical formulas according to the present invention, X is an oxygen atom or a sulfur atom.

In the present invention, R is more preferably a furyl group (preferably a 2-furyl group), and X is more preferably an oxygen atom.

Examples of the salt of the semicarbazide or the thiosemicarbazide of formula (C2) (hereinafter, referred to as the semicarbazide or the thiosemicarbazide (C2)) or the 1,3,4-oxa(thia)diazol-2-amine derivative of formula (C4) (hereinafter, referred to as the 1,3,4-oxa(thia)diazol-2-amine derivative (C4)) include pharmacologically acceptable acid-addition salts. Examples thereof include salts of inorganic acids such as hydrochloric acid, hydrobromic acid, sulfuric acid, nitric acid, phosphoric acid, and boric acid, and salts of organic acids including carboxylic acids such as formic acid, acetic acid, propionic acid, fumaric acid, malonic acid, succinic acid, maleic acid, tartaric acid, citric acid, and benzoic acid, sulfonic acids such as methanesulfonic acid and p-toluenesulfonic acid, and amino acids such as glutamic acid and aspartic acid.

Hereinafter, the method for producing the 1,3,4-oxa(thia)diazol-2-amine derivative (C4) according to the present invention will be described in detail. Starting materials and produced compounds may be in the form of salts. A compound produced through each reaction can be converted to a salt by a routine method.

The production of the 1,3,4-oxa(thia)diazol-2-amine derivative (C4) or the salt thereof according to the present invention is performed by dehydration-condensing an aldehyde of formula (C1) (hereinafter, referred to as an aldehyde (C1)) and semicarbazide or thiosemicarbazide (C2) or a salt thereof, and cyclizing the resulting semicarbazone or thiosemicarbazone of formula (C3) (hereinafter, referred to as a semicarbazone or a thiosemicarbazone (C3)), without isolation, using chloramine T.

The condensation reaction of the aldehyde (C1) with the semicarbazide or the thiosemicarbazide (C2) or the salt thereof can usually be performed in water or a polar solvent using an acid as a condensation accelerator.

The ratio between the aldehyde (C1) and the semicarbazide or the thiosemicarbazide (C2) or the salt thereof used is preferably from 1:1 to 1:1.20, more preferably from 1:1.03 to 1:1.10, in terms of a molar ratio.

The reaction temperature can usually be in the range of -78°C to the boiling point of the solvent used and is preferably from 0 to 50°C.

The atmosphere in which the reaction is performed may be an air atmosphere or may be an inert gas atmosphere (e.g., nitrogen and argon) atmosphere.

As for reaction pressure, the reaction is preferably performed under atmospheric pressure from the viewpoint of economic performance but may be performed under increased or reduced pressure conditions.

Examples of the polar solvent include alcohol solvents such as methanol, ethanol, propanol, isopropyl alcohol, and butanol, nitrile solvents such as acetonitrile, isobutyronitrile, propionitrile, and methoxyacetonitrile, and ether solvents such as tetrahydrofuran (THF) and 1,3- or 1,4-dioxane. Water or a mixed solution of water and tetrahydrofuran (THF) is more preferably used, and the amount of the solvent used is not particularly limited.

Examples of the acid for use as the condensation accelerator described above include hydrochloric acid, sulfuric acid, acetic acid, carbonic acid, p-toluenesulfonic acid, nitric acid, oxalic acid, phosphoric acid, hydrobromic acid, hydroiodic acid, sulfamic acid, and perchloric acid. Hydrochloric acid, acetic acid, or carbonic acid is preferred.

In a preferred embodiment, the reaction is performed, for example, in an aqueous solution containing sodium acetate dissolved therein.

The aldehyde (C1) may be obtained as a commercially available product or can be obtained by a method described in a literature, etc. or a method equivalent thereto. The semicarbazide or the thiosemicarbazide(C2) or the salt thereof may be obtained as a commercially available product of semicarbazide hydrochloride or thiosemicarbazide.

Subsequently, the semicarbazone or the thiosemicarbazone (C3) formed through the reaction is oxidatively cyclized, without isolation, under basic conditions in an aqueous solvent using chloramine T. Specifically, after completion of the condensation reaction, a solvent, a base, and chloramine T are added to the reaction solution, and cyclization reaction is performed in one pot.

The solvent used is not particularly limited as long as the solvent does not participate in the reaction. Examples thereof include alcohol solvents such as methanol, ethanol, propanol, isopropyl alcohol, and butanol, nitrile solvents such as acetonitrile, isobutyronitrile, propionitrile, and methoxyacetonitrile, and ether solvents such as tetrahydrofuran (THF) and 1,3-or 1,4-dioxane. Among them, tetrahydrofuran (THF) is more preferred. These solvents may be used alone or in combination, and the amount of the solvent used is not particularly limited.

Examples of the base include potassium hydroxide, potassium carbonate, potassium bicarbonate, tripotassium phosphate, sodium hydroxide, sodium carbonate, and sodium bicarbonate. Potassium carbonate is more preferred.

The amount of the base is from 2.00 to 4.00 times by mole, preferably from 2.50 to 3.00 times by mole the amount of the aldehyde.

Chloramine T means sodium p-toluenesulfonchloramide. Commercially available chloramine T trihydrate or the like can be used.

The amount of chloramine T is from 1.10 to 2.00 times by mole, preferably from 1.20 to 1.70 times by mole the amount of the aldehyde.

The reaction temperature can usually be in the range of from -78°C to the boiling point of the solvent used and is preferably from 0°C to the boiling point of the aqueous solvent used.

The reaction time is usually preferably from 5 minutes to 48 hours, more preferably from 1 to 30 hours.

After completion of the reaction, the 1,3,4-oxa(thia)diazol-2-amine derivative (C4) or the salt thereof can be separated by washing the reaction solution with a mixed solution of an aqueous solution of a reducing agent such as sodium thiosulfate, sodium sulfite, or sodium bisulfite and aqueous sodium chloride solution, then adding thereto an organic solvent such as toluene, diethyl ether, diisopropyl ether (IPE), or t-butyl methyl ether (MTBE), performing extraction operation using a mixed solution of an aqueous hydrochloric acid solution and aqueous sodium chloride solution, performing crystallization by adding a basic aqueous solution of sodium hydroxide or the like to the obtained aqueous layer, and collecting the crystals by filtration; by washing the reaction solution with a mixed solution of an aqueous solution of a reducing agent such as sodium thiosulfate, sodium sulfite, or sodium bisulfite and aqueous sodium chloride solution, then adding thereto hydrochloric acid dissolved in an organic solvent such as diethyl ether, ethyl acetate, or 1,4-dioxane, collecting the resulting solid by filtration, suspending the obtained solid in water, and adding a basic aqueous solution of sodium hydroxide or the like to the suspension, followed by collection by filtration; by adding an organic solvent such as toluene, diethyl ether, diisopropyl ether (IPE), or t-butyl methyl ether (MTBE) to the filtrate mentioned above, performing extraction operation using a mixed solution of an aqueous hydrochloric acid solution and aqueous sodium chloride solution, performing crystallization by adding a basic aqueous solution of sodium hydroxide or the like to the obtained aqueous layer, and collecting the crystals by filtration; by adding a mixed solution of an aqueous solution of a reducing agent such as sodium thiosulfate, sodium sulfite, or sodium bisulfite and aqueous sodium chloride solution to the reaction solution, and collecting the precipitated solid by filtration; by separating the organic layer from the filtrate mentioned above, adding an organic solvent such as toluene, diethyl ether, diisopropyl ether (IPE), or t-butyl methyl ether (MTBE) to the obtained organic layer, performing extraction operation using a mixed solution of an aqueous hydrochloric acid solution and aqueous sodium chloride solution, performing crystallization by adding a basic aqueous solution of sodium hydroxide or the like to the obtained aqueous layer, and collecting the crystals by filtration; by washing the reaction solution after completion of the reaction with a mixed solution of an aqueous solution of a reducing agent such as sodium thiosulfate, sodium sulfite, or sodium bisulfite and aqueous sodium chloride solution, then adding thereto an organic solvent such as toluene, diethyl ether, diisopropyl ether (IPE), or t-butyl methyl ether (MTBE), performing extraction operation using a mixed solution of an aqueous hydrochloric acid solution and aqueous sodium chloride solution, and rendering the obtained aqueous layer basic by the addition of a basic aqueous solution of sodium hydroxide or the like, followed by concentration under reduced pressure; by washing the reaction solution after completion of the reaction with a mixed solution of an aqueous solution of a reducing agent such as sodium thiosulfate, sodium sulfite, or sodium bisulfite and aqueous sodium chloride solution, then adding thereto a mixed solution of an aqueous hydrochloric acid solution and aqueous sodium chloride solution, performing extraction operation using an organic solvent such as toluene, diethyl ether, diisopropyl ether (IPE), or t-butyl methyl ether (MTBE), and washing the obtained organic layer with a basic aqueous solution of sodium bicarbonate, sodium hydroxide or the like, followed by concentration under reduced pressure; or by washing the reaction solution with a mixed solution of an aqueous solution of a reducing agent such as sodium thiosulfate, sodium sulfite, or sodium bisulfite and aqueous sodium chloride solution, then adding thereto an organic solvent such as toluene, diethyl ether, diisopropyl ether (IPE), or t-butyl methyl ether (MTBE) and hydrochloric acid dissolved in an organic solvent such as diethyl ether, ethyl acetate, or 1,4-dioxane, performing extraction operation using a mixed solution of an aqueous hydrochloric acid solution and aqueous sodium chloride solution, and performing crystallization by adding a basic aqueous solution of sodium hydroxide or the like to the obtained aqueous layer, and collecting the crystals by filtration. The 1,3,4-oxa(thia)diazol-2-amine derivative (C4) or the salt thereof may be subjected to purification operation by silica gel column chromatography.

It has previously been reported that a 1,3,4-oxa(thia)diazol-2-amine derivative (C4), for example, 5-(furan-2-yl)-1,3,4-oxadiazol-2-amine, is produced by a production method of reacting furfural with semicarbazide (C2) to obtain semicarbazone (C3), which is then isolated and then cyclized using iodine as an oxidizing agent (Non Patent Literature C1). However, problems of this method are given work due to the isolation of semicarbazone, use of highly toxic, corrosive and sublimating iodine, and low yields of the cyclization reaction.

By contrast, the method of the present invention can efficiently produce a 5-(furan-2-yl)-1,3,4-oxa(thia)diazol-2-amine derivative (C4) or a salt thereof without the operation of isolating semicarbazone and without the use of highly toxic, corrosive and sublimating iodine.

Hereinafter, the present invention will be described in detail with reference to Examples and Comparative Examples. However, the present invention is not limited by these examples by any means.

### Examples

### Example A1 Production of 2-phenyl-6-(trifluoromethoxy)quinoline-4-carboxylic acid

To a solution of 4-(trifluoromethoxy)aniline (1.81 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise to the reaction solution over 3 hours. 21 hours later, the reaction solution was cooled to room temperature, and water was added thereto, followed by extraction with toluene. The obtained organic layer was washed with water, followed by extraction with a 1 mol/L sodium hydroxide aqueous solution. The obtained aqueous layer was adjusted to pH 1 or lower by the addition of concentrated hydrochloric acid and then stirred for 1 hour. The resulting solid was collected by filtration, washed with water, and then dried under reduced pressure to obtain the title compound (1.55 g, yield: 82%) as a pale yellowish-white solid.

¹H-NMR (400 MHz, DMSO-D₆) δ: 14.2 (1H, s), 8.74 (1H, d, J=1.5 Hz), 8.33-8.30 (3H, m), 7.87 (1H, dd, J=9.2, 2.7 Hz), 7.63-7.54 (3H, m) ESI-MS (m/z): 334 (M+H)⁺

### Example A2 Production of 2-phenyl-6-(trifluoromethoxy)quinoline-4-carboxylic acid

To a solution of 4-(trifluoromethoxy)aniline (1.81 g, 10.2 mmol) in acetonitrile (5.7 mL), benzaldehyde (1.21 g, 11.4 mmol) and boron trifluoride-diethyl ether complex (357 µL, 2.84 mmol) were added, and the mixture was heated to 65°C. 1 hour later, a solution of pyruvic acid (0.500 g, 5.68 mmol) in acetonitrile (9.5 mL) was added dropwise to the reaction solution over 3 hours. 21 hours later, the reaction solution was cooled to room temperature, and water was added thereto, followed by extraction with toluene. The obtained organic layer was washed with water, followed by extraction with a 1 mol/L sodium hydroxide aqueous solution. The obtained aqueous layer was adjusted to pH 1 or lower by the addition of concentrated hydrochloric acid and then stirred for 1 hour. The resulting solid was collected by filtration, washed with water, and then dried under reduced pressure to obtain the title compound (1.62 g, yield: 86%) as a pale yellowish-white solid.

### Example A3 Production of 2-phenylquinoline-4-carboxylic acid

To a solution of aniline (0.952 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and water was added thereto, followed by extraction with toluene. The obtained organic layer was washed with water, followed by extraction with a 1 mol/L sodium hydroxide aqueous solution. The obtained aqueous layer was adjusted to pH 1 or lower by the addition of concentrated hydrochloric acid, and saturated aqueous sodium chloride solution was then added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was purified by silica gel column chromatography (hexane/ethyl acetate) to obtain the title compound (0.614 g, yield: 43%) as a pale yellow solid.

¹H-NMR (400 MHz, DMSO-D₆) δ: 14.0 (1H, s), 8.67 (1H, d, J=8.5 Hz), 8.47 (1H, s), 8.32-8.30 (2H, m), 8.18 (1H, d, J=7.9 Hz), 7.89-7.85 (1H, m), 7.74-7.70 (1H, m), 7.61-7.53 (3H, m) ESI-MS (m/z): 250 (M+H)⁺

### Example A4 Production of 6-fluoro-2-phenylquinoline-4-carboxylic acid

To a solution of 4-fluoroaniline (1.14 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (1.10 g, yield: 72%) as a pale yellow solid.

¹H-NMR (400 MHz, DMSO-D₆) δ: 14.1 (1H, s), 8.56 (1H, s), 8.48 (1H, dd, J=11.0, 2.7 Hz), 8.30-8.27 (2H, m), 8.26 (1H, dd, J=9.3, 6.0 Hz), 7.83-7.78 (1H, m), 7.61-7.53 (3H, m) ESI-MS (m/z): 268 (M+H)⁺

### Example A5 Production of 6-chloro-2-phenylquinoline-4-carboxylic acid

To a solution of 4-chloroaniline (1.30 g, 10.2 mmol) in acetonitrile (9.5 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (5.7 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed in chloroform and dried under reduced pressure to obtain the title compound (1.27 g, yield: 79%) as an ocher solid.

¹H-NMR (400 MHz, DMSO-D₆) δ: 14.1 (1H, s), 8.79 (1H, d, J=2.4 Hz), 8.56 (1H, s), 8.32-8.28 (2H, m), 8.19 (1H, d, J=9.2 Hz), 7.89 (1H, dd, J=9.2, 2.4 Hz), 7.60-7.55 (3H, m) ESI-MS (m/z): 284, 286 (M+H)⁺

### Example A6 Production of 6-bromo-2-phenylquinoline-4-carboxylic acid

To a solution of 4-bromoaniline (1.76 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran.

The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (1.32 g, yield: 71%) as a white solid.

¹H-NMR (400 MHz, DMSO-D₆) δ: 14.2 (1H, s), 8.95 (1H, d, J=2.1 Hz), 8.55 (1H, s), 8.31-8.29 (2H, m), 8.12 (1H, d, J=8.9 Hz), 7.99 (1H, dq, J=8.9, 1.1 Hz), 7.62-7.54 (3H, m) ESI-MS (m/z): 328, 330 (M)⁺

### Example A7 Production of 6-methyl-2-phenylquinoline-4-carboxylic acid

To a solution of p-toluidine (1.10 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (1.17 g, yield: 78%) as a pale yellow solid.

¹H-NMR (400 MHz, DMSO-D₆) δ: 8.44 (2H, s), 8.28 (2H, t, J=4.0 Hz), 8.09 (1H, d, J=8.5 Hz), 7.72 (1H, dd, J=8.5, 1.8 Hz), 7.59-7.54 (3H, m), 2.56 (3H, s) ESI-MS (m/z): 264 (M+H)⁺

### Example A8 Production of 6-nitro-2-phenylquinoline-4-carboxylic acid

To a solution of 4-nitroaniline (1.41 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (1.48 g, yield: 89%) as a brown solid.

¹H-NMR (400 MHz, DMSO-D₆) δ: 14.4 (1H, s), 9.70 (1H, d, J=2.4 Hz), 8.68 (1H, s), 8.54 (1H, dd, J=9.2, 2.4 Hz), 8.37-8.36 (2H, m), 8.34-8.32 (1H, m), 7.64-7.61 (3H, m) ESI-MS (m/z): 295 (M+H)⁺

### Example A9 Production of 6-hydroxy-2-phenylquinoline-4-carboxylic acid

To a solution of 4-aminophenol (1.12 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (0.907 g, yield: 60%) as a pale yellow solid.

¹H-NMR (400 MHz, DMSO-D₆) δ: 13.8 (1H, s), 10.3 (1H, s), 8.39 (1H, s), 8.24-8.21 (2H, m), 8.04 (1H, d, J=2.7 Hz), 8.02 (1H, d, J=9.2 Hz), 7.57-7.47 (3H, m), 7.40 (1H, dd, J=9.2, 2.7 Hz) ESI-MS (m/z): 266 (M+H)⁺

### Example A10 Production of 6-cyano-2-phenylquinoline-4-carboxylic acid

To a solution of 4-aminobenzonitrile (0.483 g, 4.09 mmol) in acetonitrile (2.3 mL), benzaldehyde (0.482 g, 4.54 mmol) and boron trifluoride-tetrahydrofuran complex (125 µL, 1.14 mmol) were added, and the mixture was heated to 65°C. 10 minutes later, a solution of pyruvic acid (0.200 g, 2.27 mmol) in acetonitrile (3.8 mL) was added dropwise thereto over 3 hours. 21 hours later, the reaction solution was cooled to room temperature, and saturated aqueous sodium chloride solution was added thereto, followed by extraction with tetrahydrofuran.

The organic layer was washed with saturated aqueous sodium chloride solution, dried over anhydrous magnesium sulfate, and then filtered, and the solvent was distilled off under reduced pressure. The obtained residue was purified by silica gel column chromatography (chloroform/methanol) to obtain the title compound (0.455 g, yield: 73%) as a pale yellow solid.

¹H-NMR (400 MHz, DMSO-D₆) δ: 14.4 (1H, s), 9.20 (1H, d, J=1.8 Hz), 8.62 (1H, s), 8.35-8.33 (2H, m), 8.29 (1H, d, J=8.5 Hz), 8.15 (1H, dd, J=8.5, 1.8 Hz), 7.62-7.59 (3H, m) ESI-MS (m/z): 275 (M+H)⁺

### Example All Production of 6-methoxy-2-phenylquinoline-4-carboxylic acid

To a solution of 4-methoxyaniline (1.26 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (0.824 g, yield: 52%) as a yellow solid.

¹H-NMR (400 MHz, DMSO-D₆) δ: 13.9 (1H, s), 8.47 (1H, s), 8.26-8.25 (2H, m), 8.15 (1H, d, J=2.4 Hz), 8.09 (1H, d, J=9.2 Hz), 7.59-7.49 (4H, m), 3.93 (3H, s) ESI-MS (m/z): 280 (M+H)⁺

### Example A12 Production of 6-methoxymethoxy-2-phenylquinoline-4-carboxylic acid

### (Example A12-1)

To a solution of 4-methoxymethoxyaniline (1.57 g, 10.2 mmol) in acetonitrile (5.7 mL), benzaldehyde (1.21 g, 11.4 mmol) and boron trifluoride-tetrahydrofuran complex

(313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (0.499 g, yield: 28%) as a brown solid.

### (Example A12-2)

To a solution of 4-methoxymethoxyaniline (1.57 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and toluene was added thereto, followed by extraction with a 1 mol/L sodium hydroxide aqueous solution. The obtained aqueous layer was cooled in ice, adjusted to pH on the order of 4 by the addition of concentrated hydrochloric acid, and then stirred for 0.5 hours. The resulting solid was collected by filtration, washed with water, and then dried under reduced pressure to obtain the title compound (1.14 g, yield: 65%) as a brown solid.

¹H-NMR (400 MHz, DMSO-D₆) δ: 13.9 (1H, s), 8.46 (1H, s), 8.28-8.21 (3H, m), 8.12 (1H, d, J=9.2 Hz), 7.62-7.49 (4H, m), 5.37 (2H, s), 3.45 (3H, s) ESI-MS (m/z): 310 (M+H)⁺

### Example A13 Production of 6-acetyl-2-phenylquinoline-4-carboxylic acid

To a solution of 4'-aminoacetophenone (1.38 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and water was added thereto, followed by extraction with toluene. The obtained organic layer was washed with water, followed by extraction with a 1 mol/L sodium hydroxide aqueous solution. The obtained aqueous layer was adjusted to pH 1 or lower by the addition of concentrated hydrochloric acid and then stirred for 1 hour. The resulting solid was collected by filtration, washed with water, and then dried under reduced pressure. The obtained solid was suspended and washed with chloroform and dried under reduced pressure to obtain the title compound (0.602 g, yield: 36%) as a brown solid.

¹H-NMR (400 MHz, DMSO-D₆) δ: 9.34 (1H, d, J=1.8 Hz), 8.57 (1H, s), 8.36-8.30 (3H, m), 8.24 (1H, d, J=8.7 Hz), 7.64-7.58 (3H, m), 2.73 (3H, s) ESI-MS (m/z): 292 (M+H)⁺

### Example A14 Production of 6-methoxycarbonyl-2-phenylquinoline-4-carboxylic acid

To a solution of methyl 4-aminobenzoate (1.55 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (1.38 g, yield: 79%) as a pale yellow solid.

¹H-NMR (400 MHz, DMSO-D₆) δ: 14.2 (1H, s), 9.39 (1H, t, J=1.1 Hz), 8.58 (1H, s), 8.36-8.24 (4H, m), 7.64-7.56 (3H, m), 3.96 (3H, s) ESI-MS (m/z): 308 (M+H)⁺

### Example A15 Production of 6-benzyloxy-2-phenylquinoline-4-carboxylic acid

To a solution of 4-benzyloxyaniline (2.04 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (1.32 g, yield: 66%) as a pale yellow solid.

¹H-NMR (400 MHz, DMSO-D₆) δ: 13.9 (1H, s), 8.46 (1H, s), 8.26-8.24 (3H, m), 8.10 (1H, d, J=9.5 Hz), 7.61-7.49 (6H, m), 7.45-7.34 (3H, m), 5.28 (2H, s) ESI-MS (m/z): 356 (M+H)⁺

### Example A16 Production of 6-benzyloxycarbonylamino-2-phenylquinoline-4-carboxylic acid

To a solution of benzyl (4-aminophenyl)carbamate (2.48 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (1.46 g, yield: 64%) as a faint yellow solid.

¹H-NMR (400 MHz, DMSO-D₆) δ: 13.9 (1H, s), 10.3 (1H, s), 8.92 (1H, d, J=2.4 Hz), 8.41 (1H, s), 8.27-8.24 (2H, m), 8.09 (1H, d, J=9.2 Hz), 7.90 (1H, dd, J=9.3, 2.3 Hz), 7.59-7.34 (8H, m), 5.23 (2H, s) ESI-MS (m/z): 399 (M+H)⁺

### Example A17 Production of 6-methylthio-2-phenylquinoline-4-carboxylic acid

To a solution of 4-methylthioaniline (1.42 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (1.31 g, yield: 78%) as a pale yellow solid.

¹H-NMR (400 MHz, DMSO-D₆) δ: 8.50 (1H, J=1.8 Hz), 8.48 (1H, s), 8.28-8.27 (2H, m), 8.08 (1H, d, J=8.5 Hz), 7.77-7.75 (1H, m), 7.58-7.53 (3H, m), 2.62 (3H, s) ESI-MS (m/z): 296 (M+H)⁺

### Example A18 Production of 7-fluoro-2-phenylquinoline-4-carboxylic acid

To a solution of 3-fluoroaniline (1.14 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (0.582 g, yield: 38%) as a light pink solid.

¹H-NMR (400 MHz, DMSO-D₆) δ: 14.1 (1H, s), 8.77 (1H, dd, J=9.5, 6.4 Hz), 8.47 (1H, s), 8.32-8.29 (2H, m), 7.92 (1H, dd, J=10.4, 2.7 Hz), 7.69-7.54 (4H, m) ESI-MS (m/z): 268 (M+H)⁺

### Example A19 Production of 8-fluoro-2-phenylquinoline-4-carboxylic acid

To a solution of 2-fluoroaniline (1.14 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (0.604 g, yield: 40%) as a pale yellow solid.

¹H-NMR (400 MHz, DMSO-D₆) δ: 14.2 (1H, s), 8.56 (1H, s), 8.49-8.44 (1H, m), 8.34-8.31 (2H, m), 7.74-7.68 (2H, m), 7.63-7.54 (3H, m) ESI-MS (m/z): 268 (M+H)⁺

### Example A20 Production of 7-chloro-2-phenylquinoline-4-carboxylic acid

To a solution of 3-chloroaniline (1.30 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (1.02 g, yield: 63%) as a light pink solid.

¹H-NMR (400 MHz, DMSO-D₆) δ: 14.2 (1H, s), 8.72 (1H, d, J=9.2 Hz), 8.51 (1H, s), 8.32-8.29 (2H, m), 8.23 (1H, d, J=2.1 Hz), 7.76 (1H, dd, J=9.2, 2.1 Hz), 7.62-7.54 (3H, m) ESI-MS (m/z): 284, 286 (M+H)⁺

### Example A21 Production of 8-chloro-2-phenylquinoline-4-carboxylic acid

To a solution of 2-chloroaniline (0.522 g, 4.09 mmol) in acetonitrile (2.3 mL), benzaldehyde (0.482 g, 4.54 mmol) and boron trifluoride-tetrahydrofuran complex (125 µL, 1.14 mmol) were added, and the mixture was heated to 65°C. 10 minutes later, a solution of pyruvic acid (0.200 g, 2.27 mmol) in acetonitrile (3.8 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The residue was purified by silica gel column chromatography (chloroform/methanol). The obtained crude product was suspended and washed with chloroform and dried under reduced pressure to obtain the title compound (0.232 g, yield: 36%) as a faint yellow solid.

¹H-NMR (400 MHz, DMSO-D₆) δ: 14.2 (1H, s), 8.61 (1H, dd, J=8.5, 1.2 Hz), 8.58 (1H, s), 8.40-8.38 (2H, m), 8.07 (1H, dd, J=7.3, 1.2 Hz), 7.68 (1H, dd, J=7.3, 1.2 Hz), 7.64-7.55 (3H, m) ESI-MS (m/z): 284, 286 (M+H)⁺

### Example A22 Production of 5,7-dichloro-2-phenylquinoline-4-carboxylic acid

To a solution of 3,5-dichloroaniline (1.66 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (1.40 g, yield: 78%) as a white solid.

¹H-NMR (400 MHz, DMSO-D₆) δ: 14.1 (1H, s), 8.37-8.35 (2H, m), 8.33 (1H, s), 8.24 (1H, d, J=1.8 Hz), 7.97 (1H, d, J=1.8 Hz), 7.62-7.55 (3H, m) ESI-MS (m/z): 318, 320 (M+H)⁺

### Example A23 Production of 7-methyl-2-phenylquinoline-4-carboxylic acid

To a solution of m-toluidine (1.10 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (0.554 g, yield: 37%) as a white solid.

¹H-NMR (400 MHz, DMSO-D₆) δ: 14.0 (1H, s), 8.56 (1H, d, J=8.5 Hz), 8.39 (1H, s), 8.30-8.26 (2H, m), 7.97 (1H, m), 7.60-7.51 (4H, m), 2.56 (3H, s) ESI-MS (m/z): 264 (M+H)⁺

### Example A24 Production of 8-methyl-2-phenylquinoline-4-carboxylic acid

To a solution of o-toluidine (1.10 g, 10.2 mmol) in acetonitrile (5.7 mL), benzaldehyde (1.21 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was purified by silica gel column chromatography (chloroform/methanol), and the crude product was suspended and washed with chloroform and dried under reduced pressure to obtain the title compound (0.379 g, yield: 25%) as a faint yellow solid.

¹H-NMR (400 MHz, DMSO-D₆) δ: 13.9 (1H, s), 8.47-8.44 (1H, m), 8.46 (1H, s), 8.36-8.32 (2H, m), 7.72 (1H, d, J=6.7 Hz), 7.61-7.51 (4H, m), 2.85 (3H, s) ESI-MS (m/z): 264 (M+H)⁺

### Example A25 Production of 2-(4-bromophenyl)-6-trifluoromethoxyquinoline-4-carboxylic acid

To a solution of 4-(trifluoromethoxy)aniline (1.81 g, 10.2 mmol) in acetonitrile (5.7 mL), 4-bromobenzaldehyde (2.10 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure. Water was added to the filtrate, followed by extraction with toluene. The obtained organic layer was washed with water, followed by extraction with a 1 mol/L sodium hydroxide aqueous solution. The solid obtained earlier was added to the obtained aqueous layer, and the mixture was adjusted to pH 1 or lower with concentrated hydrochloric acid and then stirred for 1 hour. The resulting solid was collected by filtration, washed with water, and then dried under reduced pressure to obtain the title compound (1.66 g, yield: 71%) as a white solid.

¹H-NMR (400 MHz, DMSO-D₆) δ: 14.3 (1H, s), 8.73 (1H, dd, J=2.7, 1.2 Hz), 8.62 (1H, s), 8.31 (1H, d, J=9.2 Hz), 8.28 (2H, d, J=8.5 Hz), 7.88 (1H, dd, J=8.9, 2.4 Hz), 7.79 (2H, d, J=8.9 Hz) ESI-MS (m/z): 412, 414 (M+H)⁺

### Example A26 Production of 2-(4-nitrophenyl)-6-trifluoromethoxyquinoline-4-carboxylic acid

To a solution of 4-(trifluoromethoxy)aniline (1.81 g, 10.2 mmol) in acetonitrile (5.7 mL), 4-nitrobenzaldehyde (1.81 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (1.52 g, yield: 71%) as a pale yellow solid.

¹H-NMR (400 MHz, DMSO-D₆) δ: 14.3 (1H, s), 8.76 (1H, d, J=1.5 Hz), 8.71 (1H, s), 8.59 (2H, d, J=8.2 Hz), 8.41 (2H, d, J=8.2 Hz), 8.36 (1H, d, J=9.2 Hz), 7.91 (1H, dd, J=9.5, 2.7 Hz) ESI-MS (m/z): 379 (M+H)⁺

### Example A27 Production of 2-(4-methoxyphenyl)-6-trifluoromethoxyquinoline-4-carboxylic acid

To a solution of 4-(trifluoromethoxy)aniline (1.81 g, 10.2 mmol) in acetonitrile (5.7 mL), 4-methoxybenzaldehyde (1.55 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and water was added thereto, followed by extraction with toluene. The obtained organic layer was washed with water, followed by extraction with a 1 mol/L sodium hydroxide aqueous solution. The obtained aqueous layer was adjusted to pH 1 or lower by the addition of concentrated hydrochloric acid and then stirred for 1 hour. The resulting solid was collected by filtration, washed with water, and then dried under reduced pressure to obtain the title compound (1.57 g, yield: 76%) as a pale yellowish-white solid.

¹H-NMR (400 MHz, DMSO-D₆) δ: 14.2 (1H, s), 8.70 (1H, dd, J=2.6, 1.1 Hz), 8.56 (1H, s), 8.29 (2H, d, J=8.5 Hz), 8.26 (1H, d, J=9.2 Hz), 7.83 (1H, dd, J=9.2, 2.7 Hz), 7.14 (2H, d, J=8.9 Hz), 3.87 (3H, s) ESI-MS (m/z): 364 (M+H)⁺

### Example A28 Production of 2-(furan-2-yl)-6-trifluoromethoxyquinoline-4-carboxylic acid

To a solution of 4-(trifluoromethoxy)aniline (1.81 g, 10.2 mmol) in acetonitrile (5.7 mL), furfural (1.09 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and water was added thereto, followed by extraction with toluene. The obtained organic layer was washed with water, followed by extraction with a 4% sodium bicarbonate aqueous solution. The obtained aqueous layer was adjusted to pH 1 or lower by the addition of concentrated hydrochloric acid and then stirred for 1 hour. The resulting solid was collected by filtration, washed with water, and then dried under reduced pressure. The obtained crude product was purified by silica gel column chromatography (hexane/ethyl acetate) to obtain the title compound (0.403 g, yield: 22%) as a pale yellowish-white solid.

¹H-NMR (400 MHz, DMSO-D₆) δ: 14.2 (1H, s), 8.73 (1H, d, J=1.2 Hz), 8.44 (1H, s), 8.22 (1H, d, J=9.2 Hz), 8.01 (1H, d, J=1.2 Hz), 7.84 (1H, dd, J=9.2, 3.1 Hz), 7.52 (1H, d, J=3.1 Hz), 6.78 (1H, dd, J=3.4, 1.2 Hz) ESI-MS (m/z): 324 (M+H)⁺

### Example A29 Production of 2-(thiophen-2-yl)-6-trifluoromethoxyquinoline-4-carboxylic acid

To a solution of 4-(trifluoromethoxy)aniline (1.81 g, 10.2 mmol) in acetonitrile (5.7 mL), 2-thiophene aldehyde (1.27 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and water was added thereto, followed by extraction with toluene. The obtained organic layer was washed with water, followed by extraction with a 1 mol/L sodium hydroxide aqueous solution. The obtained aqueous layer was adjusted to pH 1 or lower by the addition of concentrated hydrochloric acid and then stirred for 1 hour. The resulting solid was collected by filtration, washed with water, and then dried under reduced pressure to obtain the title compound (1.51 g, yield: 78%) as an ocher solid.

¹H-NMR (400 MHz, DMSO-D₆) δ: 14.3 (1H, s), 8.66 (1H, d, J=1.5 Hz), 8.57 (1H, s), 8.18 (1H, d, J=9.2 Hz), 8.15 (1H, dd, J=3.8, 1.1 Hz), 7.83 (1H, dd, J=5.0, 1.1 Hz), 7.83-7.80 (1H, m), 7.26 (1H, dd, J=4.9, 3.7 Hz) ESI-MS (m/z): 340 (M+H)⁺

### Example A30 Production of 2-(naphthalen-2-yl)-6-trifluoromethoxyquinoline-4-carboxylic acid

To a solution of 4-(trifluoromethoxy)aniline (1.81 g, 10.2 mmol) in acetonitrile (5.7 mL), 2-naphthaldehyde (1.77 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and the precipitated solid was collected by filtration. The solid was washed with acetonitrile and then dried under reduced pressure to obtain the title compound (1.70 g, yield: 78%) as a yellow solid.

¹H-NMR (400 MHz, DMSO-D₆) δ: 14.2 (1H, s), 8.92 (1H, d, J=1.8 Hz), 8.81 (1H, s), 8.76 (1H, d, J=1.8 Hz), 8.51 (1H, dd, J=8.9, 1.5 Hz), 8.36 (1H, d, J=9.2 Hz), 8.18-8.17 (1H, m), 8.12 (1H, d, J=9.2 Hz), 8.02-8.01 (1H, m), 7.89 (1H, dd, J=9.2, 1.8 Hz), 7.63-7.61 (2H, m) ESI-MS (m/z): 384 (M+H)⁺

### Example A31 Production of 3-methyl-2-phenyl-6-trifluoromethoxyquinoline-4-carboxylic acid

To a solution of 4-(trifluoromethoxy)aniline (1.56 g, 8.82 mmol) in acetonitrile (4.9 mL), benzaldehyde (1.04 g, 9.80 mmol) and boron trifluoride-tetrahydrofuran complex (270 µL, 2.45 mmol) were added, and the mixture was heated to 65°C. 10 minutes later, a solution of 2-oxobutyric acid (0.500 g, 4.90 mmol) in acetonitrile (8.2 mL) was added dropwise thereto over 3 hours. 21 hours later, the reaction solution was cooled to room temperature, and toluene was added thereto, followed by extraction with a 1 mol/L sodium hydroxide aqueous solution. The obtained aqueous layer was adjusted to pH 1 or lower by the addition of concentrated hydrochloric acid and then stirred for 1 hour. The resulting solid was collected by filtration, washed with water, and then dried under reduced pressure. The obtained crude form was suspended and washed with chloroform and dried under reduced pressure to obtain the title compound (0.640 g, yield: 38%) as a white solid.

¹H-NMR (400 MHz, DMSO-D₆) δ: 14.5 (1H, s), 8.21 (1H, d, J=9.2 Hz), 7.80 (1H, dd, J=9.2, 2.4 Hz), 7.70-7.68 (1H, m), 7.64-7.61 (2H, m), 7.55-7.51 (3H, m), 2.42 (3H, s) ESI-MS (m/z): 348 (M+H)⁺

### Example A32 Production of 2-(tert-butyl)-6-(trifluoromethoxy)quinoline-4-carboxylic acid

To a solution of 4-(trifluoromethoxy)aniline (1.81 g, 10.2 mmol) in acetonitrile (5.7 mL), pivalaldehyde (0.978 g, 11.4 mmol) and boron trifluoride-tetrahydrofuran complex (313 µL, 2.84 mmol) were added, and the mixture was heated to 65°C. 10 minutes later, a solution of pyruvic acid (0.500 g, 5.68 mmol) in acetonitrile (9.5 mL) was added dropwise thereto over 3 hours. 21 hours later, the reaction solution was cooled to room temperature, and saturated aqueous sodium chloride solution was added thereto, followed by extraction with tetrahydrofuran. The organic layer was washed with saturated aqueous sodium chloride solution, dried over anhydrous magnesium sulfate, and then filtered, and the solvent was distilled off under reduced pressure. The obtained residue was suspended and washed with chloroform and dried under reduced pressure to obtain the title compound (1.32 g, yield: 74%) as a faint yellow solid.

¹H-NMR (400 MHz, DMSO-D₆) δ: 14.1 (1H, s), 8.69 (1H, d, J=2.4 Hz), 8.19 (1H, d, J=9.2 Hz), 8.18 (1H, s), 7.80 (1H, dd, J=9.2, 2.4 Hz), 1.44 (9H, s) ESI-MS (m/z): 314 (M+H)⁺

### Comparative Example A1 Production of 2-phenyl-6-(trifluoromethoxy)quinoline-4-carboxylic acid

Benzaldehyde (300 mg, 2.83 mmol), 4-(trifluoromethoxy)aniline (551 mg, 3.11 mmol), and pyruvic acid (299 mg, 3.39 mmol) were added to water (14 mL), and the mixture was stirred. Sulfamic acid (8.2 mg, 0.085 mmol) was added thereto, and the mixture was heated to reflux. 18 hours later, the reaction solution was cooled to room temperature, followed by extraction with toluene. The organic layer was washed with water, followed by extraction with a 1 mol/L sodium hydroxide aqueous solution. The obtained aqueous layer was adjusted to pH 1 or lower by the addition of concentrated hydrochloric acid and stirred for 1 hour. The resulting solid was collected by filtration and dried under reduced pressure to obtain the title compound (44 mg, yield: 5%) as a yellow solid.

### Comparative Example A2 Production of 2-phenyl-6-(trifluoromethoxy)quinoline-4-carboxylic acid

To a solution of benzaldehyde (600 mg, 5.65 mmol) and pyruvic acid (299 mg, 3.39 mmol) in ethanol (5 mL), a solution of 4-(trifluoromethoxy)aniline (551 mg, 3.11 mmol) in ethanol (5 mL) was added, and the mixture was heated to reflux. 21 hours later, the reaction solution was cooled to room temperature, and toluene was added thereto, followed by extraction with a 1 mol/L sodium hydroxide aqueous solution. The obtained aqueous layer was adjusted to pH 1 or lower by the addition of concentrated hydrochloric acid and stirred for 1 hour. The resulting viscous solid was collected by filtration and dried under reduced pressure to obtain the title compound (0.285 g, yield: 15%) as an orange amorphous solid.

### Example A33 Production of N-(5-(furan-2-yl)-1,3,4-oxadiazol-2-yl)-2-phenyl-6-(trifluoromethoxy)quinoline-4-carboxamide

To a suspension of 2-phenyl-6-(trifluoromethoxy)quinoline-4-carboxylic acid (1.00 g, 3.00 mmol) obtained in Example 1 and 5-(furan-2-yl)-1,3,4-oxadiazol-2-amine (0.567 g, 3.75 mmol) in tetrahydrofuran (12 mL), O-(1H-benzotriazol-1-yl)-N,N,N',N'-tetramethyluronium hexafluorophosphate (HBTU) (1.54 g, 4.05 mmol) and N-methylmorpholine (NMM) (0.455 g, 4.50 mmol) were added in an argon atmosphere, and the mixture was stirred at 45°C. 24 hours later, the reaction solution was cooled to room temperature, then water was added thereto, and the mixture was stirred. 1 hour later, the resulting solid was collected by filtration, washed with water, and then dried under reduced pressure. The obtained crude form (1.63 g) was dissolved in 1-propanol (54 mL) at 90°C, and the solution was then cooled in ice and stirred for 2 hours. The resulting solid was collected by filtration and washed with 1-propanol cooled in ice, and the obtained solid was dried under reduced pressure to obtain the title compound (1.19 g, yield: 85%) as a white solid.

¹H-NMR (400 MHz, DMSO-D₆) δ: 12.9 (1H, s), 8.65 (1H, s), 8.39-8.37 (2H, m), 8.34 (1H, d, J=9.2 Hz), 8.31-8.29 (1H, m), 8.09 (1H, d, J=1.8 Hz), 7.90 (1H, dd, J=9.2, 1.8 Hz), 7.63-7.58 (3H.m), 7.33 (1H, d, J=3.7 Hz), 6.83 (1H, dd, J=3.7, 1.8 Hz) ESI-MS (m/z): 467 (M+H)⁺

### Example B1 Production of 5-(trifluoromethoxy)isatin

(1) To a solution of 2-(methoxyimino)acetic acid (5.82 g, 56.5 mmol) in acetonitrile (44 mL), carbonyldiimidazole (CDI) (8.42 g, 51.9 mmol) was added in 4 divided portions over 1 hour in an argon atmosphere, and the mixture was stirred at room temperature. 30 minutes later, 4-(trifluoromethoxy)aniline (8.00 g, 45.2 mmol) was added thereto, and the mixture was stirred at room temperature. 1.5 hours later, water was added to the reaction solution, and the mixture was stirred for 30 minutes. The resulting solid was collected by filtration, washed with water, and then dried under reduced pressure to obtain 2-(methoxyimino)-N-(4-(trifluoromethoxy)phenyl)acetamide (11.5 g, yield: 97%, purity: 99.5% (HPLC)) as a white solid.

¹H-NMR (400 MHz, DMSO-D₆) δ: 10.5 (1H, s), 7.81-7.77 (2H, m), 7.74 (1H, s), 7.36 (2H, dd, J=9.0, 0.8 Hz), 3.99 (3H, s) ESI-MS (m/z): 263 (M+H)⁺

(2) 2-(Methoxyimino)-N-(4-(trifluoromethoxy)phenyl)acetamide (10.0 g, 38.1 mmol) obtained in (1) was dissolved in concentrated sulfuric acid (48 mL), and the solution was heated to 70°C. 6.5 hours later, the reaction solution was cooled in ice, then water cooled in ice was added thereto, and the mixture was stirred for 30 minutes. The resulting solid was collected by filtration, washed with water, and then dried under reduced pressure to obtain the title compound (7.36 g, yield: 83%, purity: 98.0% (HPLC)) as an ocher solid.

¹H-NMR (400 MHz, DMSO-D₆) δ: 11.2 (1H, s), 7.61-7.58 (1H, m), 7.54 (1H, d, J=2.1 Hz), 7.00 (1H, d, J=8.5 Hz) ESI-MS (m/z): 232 (M+H)⁺

### Comparative Example B1 Production of 5-(trifluoromethoxy)isatin

(1) To a solution of 4-(trifluoromethoxy)aniline (1.00 g, 5.65 mmol) in acetonitrile (51 mL), water (5.7 mL), sodium sulfate (0.401 g, 2.82 mmol), hydrated chloral (1.49 g, 9.03 mmol), and hydroxylamine hydrochloride (0.628 g, 9.03 mmol) were added, and the mixture was heated to reflux. 20 hours later, the reaction solution was cooled to room temperature, and the solvent was distilled off under reduced pressure. Water was added to the obtained residue, and the mixture was stirred for 2 hours. The resulting solid was collected by filtration, washed with water, and then dried under reduced pressure to obtain 2-(hydroxyimino)-N-(4-(trifluoromethoxy)phenyl)acetamide (1.09 g, yield: 78%, purity: 93.0% (HPLC)) as a pale yellow solid.

¹H-NMR (400 MHz, DMSO-D₆) δ: 12.2 (1H, s), 10.4 (1H, s), 7.81-7.79 (2H, m), 7.65 (1H, s), 7.34 (2H, d, J=7.9 Hz) ESI-MS (m/z): 249 (M+H)⁺

(2) 2-(Hydroxyimino)-N-(4-(trifluoromethoxy)phenyl)acetamide (1.09 g, 5.12 mmol) obtained in (1) was dissolved in concentrated sulfuric acid (11 mL), and the solution was heated to 50°C. 23 hours later, the reaction solution was cooled in ice, then water cooled in ice was added thereto, and the mixture was stirred for 3 hours. The resulting solid was collected by filtration, washed with water, and then dried under reduced pressure to obtain the title compound (0.760 g, yield: 75%, purity: 92.2% (HPLC)) as an ocher solid.

### Example B2 Production of 2-phenyl-6-(trifluoromethoxy)quinoline-4-carboxylic acid

To a solution of 5-(trifluoromethoxy)isatin (3.00 g, 13.0 mmol) in methanol (52 mL), a 5 mol/L sodium hydroxide aqueous solution (6.5 mL, 32.4 mmol) and acetophenone (2.34 g, 19.5 mmol) were added, and the mixture was heated to reflux. 24 hours later, the reaction solution was cooled to room temperature, and the solvent was distilled off under reduced pressure. Toluene was added to the obtained residue, followed by extraction with water. The organic layer was subjected to re-extraction with a 1 mol/L sodium hydroxide aqueous solution, and the aqueous layers were combined, adjusted to pH 1 or lower with concentrated hydrochloric acid, and then stirred at room temperature for 2 hours. The resulting solid was collected by filtration, washed with water, and then dried under reduced pressure to obtain the title compound (3.84 g, yield: 89%) as a brown solid.

¹H-NMR (400 MHz, DMSO-D₆) δ: 14.2 (1H, s), 8.74 (1H, d, J=1.5 Hz), 8.33-8.30 (3H, m), 7.87 (1H, dd, J=9.2, 2.7 Hz), 7.63-7.54 (3H, m) ESI-MS (m/z): 334 (M+H)⁺

### Example B3 Production of N-(5-(furan-2-yl)-1,3,4-oxadiazol-2-yl)-2-phenyl-6-(trifluoromethoxy)quinoline-4-carboxamide

To a suspension of 2-phenyl-6-(trifluoromethoxy)quinoline-4-carboxylic acid (3.50 g, 10.5 mmol) obtained in Example 2 and 5-(furan-2-yl)-1,3,4-oxadiazol-2-amine (1.98 g, 13.1 mmol) in tetrahydrofuran (42 mL), O-(1H-benzotriazol-1-yl)-N,N,N',N'-tetramethyluronium hexafluorophosphate (HBTU) (5.38 g, 14.2 mmol) and N-methylmorpholine (NMM) (1.59 g, 15.8 mmol) were added in an argon atmosphere, and the mixture was stirred at 45°C. 24 hours later, the reaction solution was cooled to room temperature, then water was added thereto, and the mixture was stirred. 1 hour later, the resulting solid was collected by filtration, washed with water, and then dried under reduced pressure. The obtained crude form (5.71 g) was dissolved in 1-propanol (190 mL) at 90°C, and the solution was then cooled in ice and stirred for 2 hours. The resulting solid was collected by filtration, washed with 1-propanol cooled in ice, and then dried under reduced pressure to obtain the title compound (3.69 g, yield: 75%) as a white solid.

¹H-NMR (400 MHz, DMSO-D₆) δ: 12.9 (1H, s), 8.65 (1H, s), 8.39-8.37 (2H, m), 8.34 (1H, d, J=9.2 Hz), 8.31-8.29 (1H, m), 8.09 (1H, d, J=1.8 Hz), 7.90 (1H, dd, J=9.2, 1.8 Hz), 7.63-7.58 (3H, m), 7.33 (1H, d, J=3.7 Hz), 6.83 (1H, dd, J=3.7, 1.8 Hz) ESI-MS (m/z): 467 (M+H)⁺

### Example C1 Production of 5-(furan-2-yl)-1,3,4-oxadiazol-2-amine

A solution of semicarbazide hydrochloride (12.2 g, 109 mmol) and sodium acetate (8.97 g, 109 mmol) in water (104 mL) was cooled in ice, then furfural (10.0 g, 104 mmol) and tetrahydrofuran (THF) (30 mL) were added thereto, and the mixture was stirred. 1.5 hours later, tetrahydrofuran (THF) (267 mL), potassium carbonate (36.0 g, 260 mmol), and chloramine T trihydrate (36.6 g, 130 mmol) were added to the reaction solution, and the mixture was warmed to room temperature and stirred. 21 hours later, the reaction solution was washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (11.2 g, yield: 71%) as a white solid.

¹H-NMR (400 MHz, DMSO-D₆) δ: 7.92-7.91 (1H, m), 7.31 (2H, brs), 6.99 (1H, d, J=3.1 Hz), 6.71 (1H, dd, J=3.1, 1.8 Hz) ESI-MS (m/z): 152 (M+H)⁺

### Example C2 Production of 5-phenyl-1,3,4-oxadiazol-2-amine

### (Example C2-1)

A solution of semicarbazide hydrochloride (1.22 g, 10.9 mmol) and sodium acetate (0.897 g, 10.9 mmol) in water (10 mL) was cooled in ice, then benzaldehyde (1.11 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (3.67 g, 13.0 mmol) were added to the reaction solution, and the mixture was heated to reflux. 2.5 hours later, chloramine T trihydrate (1.17 g, 4.16 mmol) was further added thereto. 4 hours later, the reaction solution was cooled to room temperature and washed with a 2:1 mixed solution of a 20% sodium bisulfite aqueous solution and 30% aqueous sodium chloride solution. 4 mol/L hydrochloric acid in ethyl acetate (7.8 mL, 31.2 mol) was added to the obtained organic layer, and the mixture was stirred at room temperature. 30 minutes later, the resulting solid was collected by filtration and washed with tetrahydrofuran (THF). The obtained solid was suspended in water (30 mL), and the suspension was adjusted to pH 12 or higher by the addition of a 10 mol/L sodium hydroxide aqueous solution. After overnight stirring, the solid was collected by filtration, washed with water, and then dried under reduced pressure to obtain the title compound (0.800 g, yield: 48%) as a white solid.

### (Example C2-2)

A solution of semicarbazide hydrochloride (1.22 g, 10.9 mmol) and sodium acetate (0.897 g, 10.9 mmol) in water (10 mL) was cooled in ice, then benzaldehyde (1.11 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) was added to the obtained organic layer, and the mixture was stirred at room temperature. 30 minutes later, the resulting solid was collected by filtration and washed with tetrahydrofuran (THF). Toluene was added to the filtrate, followed by extraction with a 4:1 mixed solution of 2 mol/L hydrochloric acid and 30% aqueous sodium chloride solution. The solid collected by filtration earlier was added to the obtained aqueous layer, and the mixture was further adjusted to pH 12 or higher by the addition of a 10 mol/L sodium hydroxide aqueous solution. After overnight stirring, the solid was collected by filtration, washed with water, and then dried under reduced pressure to obtain the title compound (1.13 g, yield: 68%) as a white solid.

¹H-NMR (400 MHz, DMSO-D₆) δ: 7.81-7.80 (2H, m), 7.56-7.51 (3H, m), 7.27 (2H, brs) ESI-MS (m/z): 162 (M+H)⁺

### Example C3 Production of 5-(4-methoxyphenyl)-1,3,4-oxadiazol-2-amine

### (Example C3-1)

A solution of semicarbazide hydrochloride (1.22 g, 10.9 mmol) and sodium acetate (0.897 g, 10.9 mmol) in water (10 mL) was cooled in ice, then p-anisaldehyde (1.42 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (3.67 g, 13.0 mmol) were added to the reaction solution, and the mixture was stirred for 1 hour under cooling in ice, then stirred at room temperature for 1 hour, and then heated to reflux. 19 hours later, the reaction solution was cooled to room temperature, then a 2:1 mixed solution of a 20% sodium bisulfite aqueous solution and 30% aqueous sodium chloride solution was added thereto, and the precipitate was collected by filtration. The organic layer was separated from the filtrate, and toluene was added to the obtained organic layer, followed by extraction with a 4:1 mixed solution of 2 mol/L hydrochloric acid and 30% aqueous sodium chloride solution. The solid collected by filtration earlier was added to the obtained aqueous layer, and the mixture was further adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.49 g, yield: 75%) as a white solid.

### (Example C3-2)

A solution of semicarbazide hydrochloride (1.22 g, 10.9 mmol) and sodium acetate (0.897 g, 10.9 mmol) in water (10 mL) was cooled in ice, then p-anisaldehyde (1.42 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) and toluene were added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.59 g, yield: 80%) as a white solid.

¹H-NMR (400 MHz, DMSO-D₆) δ: 7.73 (2H, d, J=9.2 Hz), 7.13 (2H, brs), 7.08 (2H, d, J=8.5 Hz), 3.82 (3H, s) ESI-MS (m/z): 192 (M+H)⁺

### Example C4 Production of 5-(furan-3-yl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 3-furaldehyde (1.00 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (3.67 g, 13.0 mmol) were added to the reaction solution, and the mixture was stirred at room temperature. 2.5 hours later, chloramine T trihydrate (1.17 g, 4.16 mmol) was further added thereto. 4 hours later, the reaction solution was washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (0.970 g, yield: 62%) as a white solid.

¹H-NMR (400 MHz, DMSO-D₆) δ: 8.24-8.23 (1H, m), 7.86 (1H, t, J=1.8 Hz), 7.15 (2H, brs), 6.83 (1H, d, J=1.8 Hz) ESI-MS (m/z): 152 (M+H)⁺

### Example C5 Production of 5-(thiophen-2-yl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then thiophene-2-carbaldehyde (1.17 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (3.67 g, 13.0 mmol) were added to the reaction solution, and the mixture was stirred at room temperature. 2.5 hours later, chloramine T trihydrate (1.17 g, 4.16 mmol) was further added thereto. 4 hours later, the reaction solution was washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (0.895 g, yield: 51%) as a white solid.

¹H-NMR (400 MHz, DMSO-D₆) δ: 7.76 (1H, dd, J=5.2, 1.2 Hz), 7.51 (1H, dd, J=3.7, 1.2 Hz), 7.28 (2H, brs), 7.20 (1H, dd, J=5.2, 3.7 Hz) ESI-MS (m/z): 168 (M+H)⁺

### Example C6 Production of 5-(naphthalen-2-yl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 2-naphthaldehyde (1.63 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (3.67 g, 13.0 mmol) were added to the reaction solution, and the mixture was heated to reflux. 5 hours later, chloramine T trihydrate (1.17 g, 4.16 mmol) was further added thereto. 5.5 hours later, the reaction solution was cooled to room temperature, then a 2:1 mixed solution of a 20% sodium bisulfite aqueous solution and 30% aqueous sodium chloride solution was added thereto, and the precipitate was collected by filtration. The organic layer was separated from the filtrate, and toluene was added to the obtained organic layer, followed by extraction with a 4:1 mixed solution of 2 mol/L hydrochloric acid and 30% aqueous sodium chloride solution. The solid collected by filtration earlier was added to the obtained aqueous layer, and the mixture was further adjusted to pH 12 or higher by the addition of a 10 mol/L sodium hydroxide aqueous solution. After overnight stirring, the solid was collected by filtration, washed with water, and then dried under reduced pressure to obtain the title compound (1.26 g, yield: 57%) as a white solid.

¹H-NMR (400 MHz, DMSO-D₆) δ: 8.31 (1H, d, J=1.2 Hz), 8.09-8.06 (2H, m), 8.00-7.99 (1H, m), 7.95 (1H, dd, J=8.5, 1.2 Hz), 7.63-7.59 (2H, m), 7.33 (2H, brs) ESI-MS (m/z): 212 (M+H)⁺

### Example C7 Production of (E)-5-styryl-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then trans-cinnamaldehyde (1.38 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (3.67 g, 13.0 mmol) were added to the reaction solution, and the mixture was heated to reflux. 2.5 hours later, chloramine T trihydrate (1.17 g, 4.16 mmol) was further added thereto. 4 hours later, the reaction solution was cooled to room temperature and washed with a 2:1 mixed solution of a 20% sodium bisulfite aqueous solution and 30% aqueous sodium chloride solution. 4 mol/L hydrochloric acid in ethyl acetate (7.8 mL, 31.2 mol) was added to the obtained organic layer, and the mixture was stirred at room temperature. 1 hour later, the resulting solid was collected by filtration and washed with tetrahydrofuran (THF). The obtained solid was suspended in water (30 mL), and the suspension was adjusted to pH 12 or higher by the addition of a 10 mol/L sodium hydroxide aqueous solution. After overnight stirring, the solid was collected by filtration, washed with water, and then dried under reduced pressure to obtain the title compound (0.865 g, yield: 44%) as a pale yellow solid.

¹H-NMR (400 MHz, DMSO-D₆) δ: 7.69-7.68 (2H, m), 7.43-7.33 (3H, m), 7.26 (2H, brs), 7.17 (1H, d, J=16.5 Hz), 7.09 (1H, d, J=16.5 Hz) ESI-MS (m/z): 188 (M+H)⁺

### Example C8 Production of 5-ethyl-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then propionaldehyde (0.605 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.84 g, 17.2 mmol) were added to the reaction solution, and the mixture was stirred at room temperature for 1 hour and then heated to reflux. 2 hours later, the reaction solution was cooled to room temperature and washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution, and this solution was then concentrated under reduced pressure. The obtained residue was purified by silica gel column chromatography (chloroform/methanol) to obtain the title compound (0.485 g, yield: 41%) as a white solid.

¹H-NMR (400 MHz, DMSO-D₆) δ: 6.81 (2H, brs), 2.63 (2H, q, J=7.7 Hz), 1.17 (3H, t, J=7.7 Hz) ESI-MS (m/z): 114 (M+H)⁺

### Example C9 Production of 5-(tert-butyl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then pivalaldehyde (0.897 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.84 g, 17.2 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (0.826 g, yield: 56%) as a white solid.

¹H-NMR (400 MHz, DMSO-D₆) δ: 6.83 (2H, brs), 1.27 (9H, s) ESI-MS (m/z): 142 (M+H)⁺

### Example C10 Production of 5-cyclohexyl-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then cyclohexacarbaldehyde (1.17 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) and toluene were added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.04 g, yield: 60%) as a white solid.

¹H-NMR (400 MHz, DMSO-D₆) δ: 6.81 (2H, brs), 2.72-2.69 (1H, m), 1.92-1.88 (2H, m), 1.76-1.69 (2H, m), 1.63-1.61 (1H, m), 1.47-1.17 (5H, m) ESI-MS (m/z): 168 (M+H)⁺

### Example C11 Production of 5-benzyl-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 2-phenylacetaldehyde (1.25 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.84 g, 17.2 mmol) were added to the reaction solution, and the mixture was stirred at room temperature for 30 minutes and then heated to reflux. 2 hours later, the reaction solution was cooled to room temperature and washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (0.541 g, yield: 30%) as a white solid.

¹H-NMR (400 MHz, DMSO-D₆) δ: 7.35-7.34 (2H, m), 7.28-7.26 (3H, m), 6.89 (2H, brs), 4.03 (2H, s) ESI-MS (m/z): 176 (M+H)⁺

### Example C12 Production of 5-(4-chlorophenyl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 4-chlorobenzaldehyde (1.46 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.84 g, 17.2 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) was added to the obtained organic layer, and the mixture was stirred at room temperature. 30 minutes later, the resulting solid was collected by filtration and washed with tetrahydrofuran (THF). Toluene was added to the filtrate, followed by extraction with a 4:1 mixed solution of 2 mol/L hydrochloric acid and 30% aqueous sodium chloride solution. The solid collected by filtration earlier was added to the obtained aqueous layer, and the mixture was further adjusted to pH 12 or higher by the addition of a 10 mol/L sodium hydroxide aqueous solution. After overnight stirring, the solid was collected by filtration, washed with water, and then dried under reduced pressure to obtain the title compound (1.25 g, yield: 61%) as a white solid.

¹H-NMR (400 MHz, DMSO-D₆) δ: 7.81-7.78 (2H, m), 7.62-7.59 (2H, m), 7.31 (2H, brs) ESI-MS (m/z): 196, 198 (M+H)⁺

### Example C13 Production of 5-(3-methoxyphenyl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 3-methoxybenzaldehyde (1.42 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) and toluene were added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.20 g, yield: 60%) as a white solid.

¹H-NMR (400 MHz, DMSO-D₆) δ: 7.45 (1H, t, J=7.9 Hz), 7.38-7.36 (1H, m), 7.30-7.28 (1H, m), 7.26 (2H, brs), 7.11-7.06 (1H, m) 3.82 (3H, s) ESI-MS (m/z): 192 (M+H)⁺

### Example C14 Production of 5-(2-methoxyphenyl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 2-methoxybenzaldehyde (1.42 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) and toluene were added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.39 g, yield: 70%) as a white solid.

¹H-NMR (400 MHz, DMSO-D₆) δ: 7.64 (1H, dd, J=7.3, 1.8 Hz), 7.53-7.48 (1H, m), 7.20 (1H, d, J=8.5 Hz), 7.12 (2H, brs), 7.09-7.04 (1H, m), 3.85 (3H, s) ESI-MS (m/z): 192 (M+H)⁺

### Example C15 Production of 5-(2,4-dimethoxyphenyl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 2,4-dimethoxybenzaldehyde (1.73 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.84 g, 17.2 mmol) were added to the reaction solution, and the mixture was stirred at room temperature for 1 hour and then heated to reflux. 2 hours later, the reaction solution was cooled to room temperature and washed with a 2:1 mixed solution of a 20% sodium bisulfite aqueous solution and 30% aqueous sodium chloride solution. 4 mol/L hydrochloric acid in ethyl acetate (7.8 mL, 31.2 mol) was added to the obtained organic layer, and the mixture was stirred at room temperature. 1 hour later, the resulting solid was collected by filtration and washed with tetrahydrofuran (THF). Toluene was added to the filtrate, followed by extraction with a 4:1 mixed solution of 2 mol/L hydrochloric acid and 30% aqueous sodium chloride solution. The solid collected by filtration earlier was added to the obtained aqueous layer, and the mixture was further adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.63 g, yield: 71%) as a white solid.

¹H-NMR (400 MHz, DMSO-D₆) δ: 7.56 (1H, d, J=8.5 Hz), 7.02 (2H, brs), 6.70 (1H, d, J=2.4 Hz), 6.65 (1H, dd, J=8.5, 2.4 Hz), 3.84 (3H, s), 3.83 (3H, s) ESI-MS (m/z): 222 (M+H)⁺

### Example C16 Production of 5-(p-tolyl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 4-methylbenzaldehyde (1.25 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) was added to the obtained organic layer, and the mixture was stirred at room temperature. 30 minutes later, the resulting solid was collected by filtration and washed with tetrahydrofuran (THF). Toluene was added to the filtrate, followed by extraction with a 4:1 mixed solution of 2 mol/L hydrochloric acid and 30% aqueous sodium chloride solution. The solid collected by filtration earlier was added to the obtained aqueous layer, and the mixture was further adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.38 g, yield: 76%) as a white solid.

¹H-NMR (400 MHz, DMSO-D₆) δ: 7.68 (2H, d, J=7.9 Hz), 7.34 (2H, d, J=7.9 Hz), 7.20 (2H, brs), 2.36 (3H, s) ESI-MS (m/z): 176 (M+H)⁺

### Example C17 Production of 5-(2,3-dihydrobenzofuran-5-yl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 2,3-dihydrobenzofuran-5-carbaldehyde (1.54 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) and toluene were added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.72 g, yield: 81%) as a white solid.

¹H-NMR (400 MHz, DMSO-D₆) δ: 7.66 (1H, d, J=1.8 Hz), 7.53 (1H, dd, J=8.5, 1.8 Hz), 7.09 (2H, s), 6.89 (1H, d, J=8.5 Hz), 4.60 (2H, t, J=8.9 Hz), 3.24 (2H, t, J=8.9 Hz) ESI-MS (m/z): 204 (M+H)⁺

### Example C18 Production of 5-nonyl-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then decanal (1.63 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.84 g, 17.2 mmol) were added to the reaction solution, and the mixture was stirred at room temperature for 30 minutes and then heated to reflux. 2 hours later, the reaction solution was cooled to room temperature and washed with a 2:1 mixed solution of a 20% sodium bisulfite aqueous solution and 30% aqueous sodium chloride solution. Toluene was added to the obtained organic layer, and the mixture was then washed with a 4:1 mixed solution of 2 mol/L hydrochloric acid and 30% aqueous sodium chloride solution. The organic layer was further washed with a saturated aqueous solution of sodium bicarbonate, and the solvent was then distilled off under reduced pressure. The obtained residue was purified by silica gel column chromatography (chloroform/methanol) to obtain the title compound (0.874 g, yield: 40%) as a white solid.

¹H-NMR (400 MHz, DMSO-D₆) δ: 6.81 (2H, brs), 2.60 (2H, t, J=7.3 Hz), 1.62-1.55 (2H, m), 1.29-1.26 (12H, m), 0.86 (3H, t, J=7.3 Hz) ESI-MS (m/z): 212 (M+H)⁺

### Example C19 Production of 5-(naphthalen-1-yl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 1-naphthaldehyde (1.63 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) was added to the obtained organic layer, and the mixture was stirred at room temperature. 30 minutes later, the resulting solid was collected by filtration and washed with tetrahydrofuran (THF). Toluene was added to the filtrate, followed by extraction with a 4:1 mixed solution of 2 mol/L hydrochloric acid and 30% aqueous sodium chloride solution. The solid collected by filtration earlier was added to the obtained aqueous layer, and the mixture was further adjusted to pH 12 or higher by the addition of a 10 mol/L sodium hydroxide aqueous solution. After overnight stirring, the solid was collected by filtration, washed with water, and then dried under reduced pressure to obtain the title compound (1.19 g, yield: 54%) as a white solid.

¹H-NMR (400 MHz, DMSO-D₆) δ: 9.13 (1H, dd, J=8.7, 0.9 Hz), 8.10 (1H, d, J=8.2 Hz), 8.05 (1H, dd, J=7.6, 1.4 Hz), 7.98 (1H, dd, J=7.3, 1.4 Hz), 7.72-7.62 (3H, m), 7.37 (2H, m) ESI-MS (m/z): 212 (M+H)⁺

### Example C20 Production of 5-(2-methylprop-1-en-1-yl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 3-methylbut-2-enal (0.876 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 4.5 hours later, the reaction solution was heated to reflux. 18 hours later, chloramine T trihydrate (1.17 g, 4.2 mmol) was added thereto. 1 hour later, the reaction solution was cooled to room temperature and washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the solid was collected by filtration, washed with water, and then dried under reduced pressure to obtain the title compound (0.837 g, yield: 58%) as a white solid.

¹H-NMR (400 MHz, DMSO-D₆) δ: 6.99 (2H, brs), 5.98-5.97 (1H, m), 2.06 (3H, s), 1.91 (3H, d, J=1.2 Hz) ESI-MS (m/z): 140 (M+H)⁺

### Example C21 Production of 5-propyl-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then butyraldehyde (0.751 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.84 g, 17.2 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution, and this solution was then concentrated under reduced pressure. The obtained residue was purified by silica gel column chromatography (chloroform/methanol) to obtain the title compound (0.626 g, yield: 47%) as a white solid.

¹H-NMR (400 MHz, DMSO-D₆) δ: 6.83 (2H, brs), 2.59 (2H, t, J=7.3 Hz), 1.64-1.60 (2H, m), 0.92 (3H, t, J=7.6 Hz) ESI-MS (m/z): 128 (M+H)⁺

### Example C22 Production of 5-(4-nitrophenyl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 4-nitrobenzaldehyde (1.57 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.84 g, 17.2 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) and toluene were added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (0.398 g, yield: 19%) as a yellow solid.

¹H-NMR (400 MHz, DMSO-D₆) δ: 8.37 (2H, d, J=8.5 Hz), 8.03 (2H, d, J=8.5 Hz), 7.55 (2H, brs) ESI-MS (m/z): 207 (M+H)⁺

### Example C23 Production of 4-(5-amino-1,3,4-oxadiazol-2-yl)phenol

A solution of semicarbazide hydrochloride (1.22 g, 10.9 mmol) and sodium acetate (0.897 g, 10.9 mmol) in water (10 mL) was cooled in ice, then 4-hydroxybenzaldehyde (1.27 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. Toluene was added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution and then concentrated under reduced pressure. The obtained residue was purified by silica gel column chromatography (chloroform/methanol) to obtain the title compound (0.353 g, yield: 19%) as a pale yellow solid.

¹H-NMR (400 MHz, DMSO-D₆) δ: 10.6 (1H, brs), 7.62-7.61 (2H, m), 7.07 (2H, brs), 6.89-6.88 (2H, m) ESI-MS (m/z): 178 (M+H)⁺

### Example C24 Production of 5-(2,6-dimethoxyphenyl)-1,3,4-oxadiazol-2-amine

A solution of semicarbazide hydrochloride (1.22 g, 10.9 mmol) and sodium acetate (0.897 g, 10.9 mmol) in water (10 mL) was cooled in ice, then 2,6-dimethoxybenzaldehyde (1.73 g, 10.4 mmol) and tetrahydrofuran (THF) (3.0 mL) were added thereto, and the mixture was stirred. 1.5 hours later, tetrahydrofuran (THF) (27 mL), potassium carbonate (3.60 g, 26.0 mmol), and chloramine T trihydrate (4.11 g, 14.6 mmol) were added to the reaction solution, and the mixture was stirred at room temperature. 3 hours later, the reaction solution was washed with a 2:1 mixed solution of a 20% sodium bisulfite aqueous solution and 30% aqueous sodium chloride solution. 4 mol/L hydrochloric acid in ethyl acetate (7.8 mL, 31.2 mol) and toluene were added to the obtained organic layer, followed by extraction with a 4:1 mixed solution of 2 mol/L hydrochloric acid and 30% aqueous sodium chloride solution. The obtained aqueous layer was adjusted to pH 12 or higher by the addition of a 10 mol/L sodium hydroxide aqueous solution. After overnight stirring, the resulting solid was collected by filtration, washed with water, and then dried under reduced pressure to obtain the title compound (1.39 g, yield: 70%) as a pale yellow solid.

¹H-NMR (400 MHz, DMSO-D₆) δ: 7.49 (1H, dd, J=8.5, 7.9 Hz), 6.98 (2H, brs), 6.76 (2H, d, J=8.5 Hz), 3.74 (6H, s) ESI-MS (m/z): 222 (M+H)⁺

### Comparative Example C1 Production of 5-(furan-2-yl)-1,3,4-oxadiazol-2-amine

5-(Furan-2-yl)-1,3,4-oxadiazol-2-amine was produced by a conventional production method, and its yield was compared with the production method of the present invention. Specifically, to a solution of semicarbazide hydrochloride (55.8 mg, 0.500 mmol) and sodium acetate (41.0 mg, 0.500 mmol) in water (1.0 mL), a solution of furfural (48.0 mg, 0.500 mmol) in methanol (1.0 mL) was added, and the mixture was stirred at room temperature. 30 minutes later, the reaction solution was concentrated under reduced pressure. The obtained residue was suspended in 1,4-dioxane (5.0 mL), and potassium carbonate (207 mg, 1.50 mmol) and iodine (152 mg, 0.600 mmol) were added to the suspension. The reaction container was hermetically sealed and heated to 80°C. 4 hours later, the reaction solution was cooled to room temperature, and a 5% sodium thiosulfate aqueous solution was added thereto, followed by extraction with a solution of dichloromethane/methanol = 10/1. The obtained organic layer was dried over anhydrous sodium sulfate and then filtered, and the solvent was distilled off under reduced pressure. The obtained residue was purified by silica gel column chromatography (chloroform/methanol) to obtain the title compound (12.6 mg, yield: 17%) as a white solid.

The yield of the conventional production method was low as compared with the production method of the present invention.

## Claims

1. A method for producing a quinolinecarboxylic acid derivative of the following formula (A4): 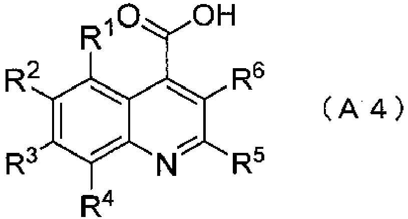 wherein R¹, R², R³ and R⁴ are the same or different and each represent a hydrogen atom, a halogen atom, a lower alkyl group, a nitro group, a hydroxy group, a cyano group, an optionally substituted phenyl group, an optionally substituted 5- or 6-membered heterocyclic group, a lower alkoxy group, a halo-lower alkoxy group, a lower alkoxy-lower alkoxy group, a lower alkyl-carbonyl group, a lower alkoxy-carbonyl group, a benzyloxy group, a trityloxy group, a t-butyldimethylsilyloxy group, a dilower alkyl-amino group, a t-butoxycarbonylamino group, a benzyloxycarbonylamino group, a 2-nitrobenzenesulfonylamino group or a lower alkyl-thio group;
R⁵ represents a hydrogen atom, a lower alkyl group, a cyclo-lower alkyl group, an optionally substituted phenyl group, an optionally substituted 5- or 6-membered heterocyclic group, a naphthyl group, a 1,3-benzodioxolyl group, a styryl group, or -CR^{5a}OR^{5b}CH₂OR^{5c}, wherein R^{5a} represents a methyl group or a t-butyl group, and R^{5b} and
R^{5c} are the same or different and each represent a hydrogen atom, a lower alkyl group, a lower alkanoyl group, a t-butyldimethylsilyl group, a 2-(trimethylsilyl)ethoxymethyl group, a benzyloxymethyl group, a phenyl group or a benzyl group optionally having a substituent on the ring, a lower alkenyl group or a lower alkoxy-methyl group; and
R⁶ represents a hydrogen atom, a lower alkyl group, a hydroxy group, an aryl group or a halogen atom,
or a salt thereof, the method comprising
reacting an aniline of the following formula (A1): 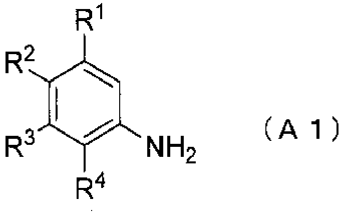 wherein R¹, R², R³ and R⁴ are as defined above,
with an aldehyde of the following formula (A2): 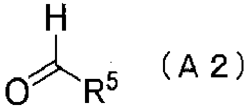
wherein R⁵ is as defined above,
in the presence of boron trifluoride-tetrahydrofuran complex or boron trifluoride-diethyl ether complex; and subsequently reacting the resulting compound with an α-keto acid of the following formula (A3): 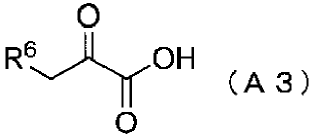 wherein R⁶ is as defined above.

2. The method according to claim 1, wherein the aniline of formula (A1) is 4-(trifluoromethoxy)aniline, the aldehyde of formula (A2) is benzaldehyde, and the α-keto acid of formula (A3) is pyruvic acid.

3. A method for producing a quinolinecarboxamide derivative of the following formula (A6): 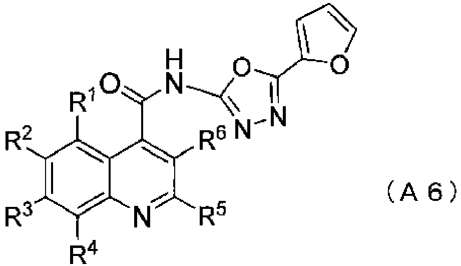 wherein R¹, R², R³, R⁴, R⁵ and R⁶ are as defined above, or a salt thereof, the method comprising
reacting a quinolinecarboxylic acid derivative of formula (A4) or a salt thereof produced by a method according to claim 1 or 2 with 5-(furan-2-yl)-1,3,4-oxadiazol-2-amine of the following formula (A5):

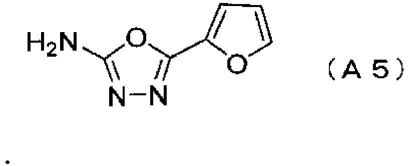

4. A method for producing an isatin derivative of the following formula (B4): 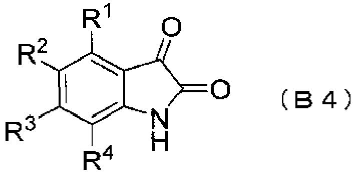 wherein R¹, R², R³ and R⁴ are the same or different and each represent a hydrogen atom, a halogen atom, a lower alkyl group, a nitro group, a hydroxy group, a cyano group, an optionally substituted phenyl group, an optionally substituted 5- or 6-membered heterocyclic group, a lower alkoxy group, a halo-lower alkoxy group, a lower alkoxy-lower alkoxy group, a lower alkylcarbonyl group, a lower alkoxy-carbonyl group, a benzyloxy group, a trityloxy group, a t-butyldimethylsilyloxy group, a dilower alkyl-amino group, a t-butoxycarbonylamino group, a benzyloxycarbonylamino group, a 2-nitrobenzenesulfonylamino group or a lower alkyl-thio group,
or a salt thereof, the method comprising
reacting an aniline of the following formula (B1): 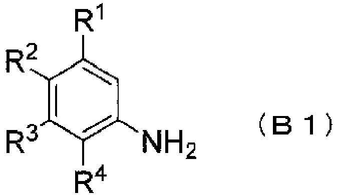 wherein R¹, R², R³ and R⁴ are as defined above,
with a 2-(alkoxyimino)acetic acid of the following formula (B2): 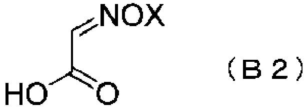
wherein X represents a lower alkyl group,
to prepare a 2-(alkoxyimino)-acetamide of the following formula (B3): 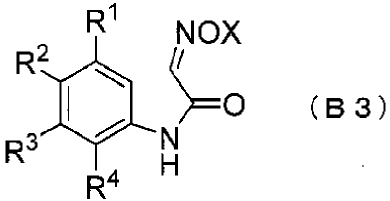
wherein R¹, R², R³, R⁴ and X are as defined above, and
heating this compound in concentrated sulfuric acid.

5. The method according to claim 4, wherein the aniline of formula (B1) is 4-(trifluoromethoxy)aniline.

6. A method for producing a quinolinecarboxylic acid derivative of the following formula (B6): 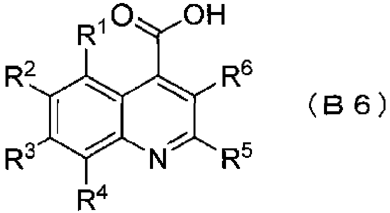 wherein R¹, R², R³ and R⁴ are as defined above; R⁵ represents a hydrogen atom, a lower alkyl group, a cyclo-lower alkyl group, an optionally substituted phenyl group, an optionally substituted 5- or 6-membered heterocyclic group, a naphthyl group, a 1,3-benzodioxolyl group, a styryl group, or -CR^{5a}OR^{5b}CH₂OR^{5c}, wherein R^{5a} represents a methyl group or a t-butyl group, and R^{5b} and R^{5c} are the same or different and each represent a hydrogen atom, a lower alkyl group, a lower alkanoyl group, a t-butyldimethylsilyl group, a 2-(trimethylsilyl)ethoxymethyl group, a benzyloxymethyl group, a phenyl group or a benzyl group optionally having a substituent on the ring, a lower alkenyl group or a lower alkoxy-methyl group; and R⁶ represents a hydrogen atom, a lower alkyl group, a hydroxy group, an aryl group or a halogen atom,
or a salt thereof, the method comprising
reacting an isatin derivative of formula (B4) or a salt thereof produced by a method according to claim 4 or 5 with a ketone of the following formula (B5): 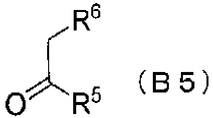
wherein R⁵ and R⁶ are as defined above,
in the presence of a base.

7. The method according to claim 6, wherein the ketone of formula (B5) is acetophenone.

8. The method according to claim 6 or 7, wherein the base is sodium hydroxide.

9. A method for producing a quinolinecarboxamide derivative of the following formula (B8): 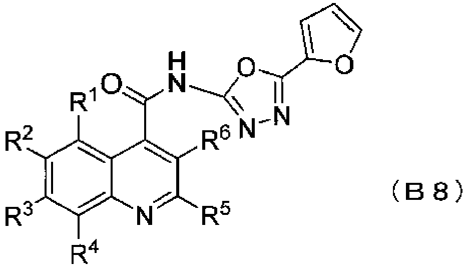 wherein R¹, R², R³, R⁴, R⁵ and R⁶ are as defined above or a salt thereof, the method comprising
reacting a quinolinecarboxylic acid derivative of formula (B6) or a salt thereof produced by a method according to any of claims 6 to 8 with 5-(furan-2-yl)-1,3,4-oxadiazol-2-amine of the following formula (B7):

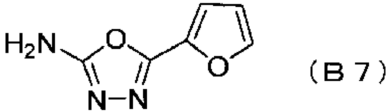

10. A method for producing a 1,3,4-oxa(thia)diazol-2-amine derivative of the following formula (C4): 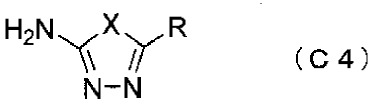 wherein R represents a hydrogen atom, a lower alkyl group, a lower alkenyl group, a cyclo-lower alkyl group, an optionally substituted phenyl group, an optionally substituted 5- or 6-membered heterocyclic group, a benzyl group, a naphthyl group or a styryl group, and X represents an oxygen atom or a sulfur atom,
or a salt thereof, the method comprising
reacting an aldehyde of the following formula (C1): 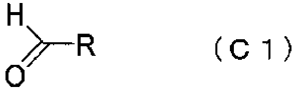 wherein R is as defined above,
with semicarbazide or thiosemicarbazide of the following formula (C2): 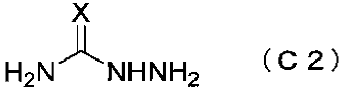
wherein X is as defined above
or a salt thereof, and
cyclizing the resulting semicarbazone or thiosemicarbazone of the following formula (C3): 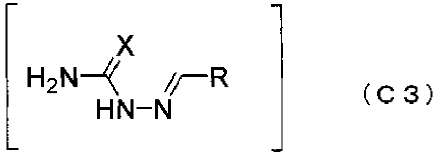 wherein R and X are as defined above,
without isolation, using chloramine T.

11. The method according to claim 10, wherein the aldehyde of formula (C1) is furfural, and the semicarbazide or the thiosemicarbazide of formula (C2) or the salt thereof is semicarbazide or a salt thereof.
